# EUROPEAN PATENT SPECIFICATION

(11) **EP 4 417 169 B1**
(45) Date of publication and mention of the grant of the patent: **14.10.2026**
(21) Application number: 24181200.7
(22) Date of filing: 29.06.2016
(51) Int. Cl.: A61F 2/07, A61F 2/954

(54) **LOCKING ASSEMBLY FOR COUPLING GUIDEWIRE TO DELIVERY SYSTEM**
VERRIEGELUNGSANORDNUNG ZUR KOPPLUNG EINES FÜHRUNGSDRAHTS AN EIN ABGABESYSTEM
ENSEMBLE DE VERROUILLAGE POUR COUPLER UN FIL-GUIDE À UN SYSTÈME DE DISTRIBUTION

(30) Priority: 30.06.2015 US 201562187103 P
(43) Date of publication of application: 21.08.2024
(62) Document number(s) of the earlier application(s) in accordance with Art. 76 EPC: 16790254.3 / 3 139 860
(73) Proprietor: Endologix LLC, Irvine, CA 92618 (US)
(72) Inventor: LEE, Rolando, Irvine, CA 92618 (US); WELK, Craig, Irvine, CA 92618 (US); TRAN, Bryant, Irvine, CA 92618 (US); CHU, Kevin, Irvine, CA 92618 (US)
(74) Representative: Forresters IP LLP

(56) References cited:
- EP-B1- 1 680 170
- US-A1- 2009 171 429
- US-A1- 2011 224 772
- US-A1- 2014 194 970

## Description

This application claims the benefit of U.S. Provisional Application no. 62/187103, filed June 30, 2015.

### BACKGROUND

### Technical Field

The present disclosure relates to devices and methods for reversibly coupling a guidewire or other elongate structure to a delivery system.

### Description of the Related Art

Some surgical procedures require multiple guidewires, for example to facilitate delivery of an implant to a branched vessel or maintain access in a branched vessel. During these procedures, an implant can be delivered via a delivery system through a first branch vessel (e.g., ipsilateral iliac artery) and access can be provided through a second branch vessel (e.g., contralateral iliac artery) using a catheter, guidewire, or otherwise. However, existing techniques for providing access to the branch vessel involve many steps and may increase the size of the delivery system, thus making the procedure more difficult to perform.

US 2009/171429 A1 discloses an endovascular delivery system including a bifurcated prosthesis disposed on a delivery device and a snare catheter having a snare opening system.

US 2011/224772 A1 discloses a method for deploying an endoluminal vascular prosthesis using a deployment catheter that has at least a main graft portion and a first branch graft portion.

EP1680170 B1 discloses a catheter system and method for stenting a vessel at a bifurcation or sidebranch of the vessel.

### SUMMARY

The invention is defined by the claims.

Certain embodiments described herein are directed to systems, methods and apparatuses for treating endovascular aneurysms or other endovascular defects.

However, it will be appreciated that the systems, methods and apparatuses may have application to other fields. In some embodiments, the defects being treated may include, but are not limited to, abdominal aortic aneurysms, subclavian aneurysms, and thoracic aortic aneurysms, to name a few.

As mentioned above, certain surgical procedures, such as those for treating aortic aneurysms may involve the use of multiple guidewires to maintain access through multiple vessels. However, managing multiple guidewires can be difficult, for example because a contralateral guidewire may be inadvertently withdrawn when a sheath or other tubular structure is withdrawn over the contralateral guidewire. Existing systems may utilize a hollow guidewire or other tubular structure to couple a contralateral portion of the delivery system to an ipsilateral portion of the delivery system or to facilitate advancement of a contralateral guidewire to the target vessel. However, the hollow guidewire or other elongate structure increases a diameter of the contralateral portion of the delivery system and increases the number of steps involved in providing and/or removing contralateral access. Thus, it may be desirable to provide a deliver^{y} system in which the contralateral guidewire is directly secured to the ipsilateral portion of the delivery system to both reduce the diameter of the contralateral portion of the deliver^{y} system and/or reduce the number of steps to provide and/or remove contralateral access. Reducing the diameter of the contralateral portion reduces the size of the contralateral access.

Other devices and techniques use gate cannulation to access the contralateral limb of the stent graft above the bifurcation, which can be challenging and time consuming. Certain aspects of the present disclosure allow for easier deployment of the contralateral limb by providing a precannulated stent graft through the contralateral limb. The present disclosure can also allow the use of a larger guidewire (e.g., 0.035 in.). Precannulation of the contralateral limb eliminates gate cannulation, thereby simplifying the graft placement procedure.

Certain aspects of this disclosure are directed toward a locking assembly for releasably coupling a guidewire to a delivery catheter such that the guidewire can be released from the delivery catheter. In certain aspects of the locking assembly herein, a housing has a proximal end, a distal end, and a lateral wall portion. A recess extends at least partially through the lateral wall of the housing. A first lumen extends from the proximal end of the housing to the distal end of the housing along a longitudinal axis. A second lumen extends from the distal end of the housing, the diameter of the second lumen being less than the diameter of the first lumen. The second lumen is configured to receive and retain a guidewire.

Optionally, the locking assembly includes an elastomeric member that is retained in the recess. The elastomeric member can be configured to retain the guidewire when the guidewire extends into the second lumen. At least a portion of the elastomeric member can be substantially flush with an outer surface of the lateral wall of the housing. The elastomeric member can have an opening that at least partially aligns with the second lumen. The opening of the elastomeric member can have a diameter that is smaller than the diameter of the second lumen of the locking assembly to help retain a guidewire extending through the second lumen.

In certain aspects, the locking assembly has a protruding portion extending along at least a portion of the outer periphery of a distal portion of the housing to provide strain relief to the guidewire that extends distally from the second lumen. The second lumen can be positioned between the first lumen and the protruding portion.

Certain aspects of the present disclosure are directed toward a system utilizing the above-described locking assembly. The locking assembly can be fixed to a first elongate member. The system can also include a second elongate member configured to be retained by the second lumen alone or the second lumen in combination with the elastomeric member.

Certain aspects of the disclosure are directed toward a locking assembly that couples a contralateral guidewire to an ipsilateral catheter. The locking assembly has an anchoring portion configured to engage the ipsilateral catheter. The locking assembly has an interlock portion configured to retain a distal portion of the contralateral guidewire when the contralateral guidewire is advanced or retracted unless a vertical force between about 0.01 and about 6.0 lbf (e.g., between about 0.01 and about 0.5 lbf, between about 0.25 and about 0.75 lbf, between about 0.5 lbf and about 1.0 lbf, between about 0.75 lbf and about 1.15 lbf, between about 1.0 lbf and about 2.0 lbf, between about 1.5 lbf and about 2.5 lbf, between about 2.0 lbf and about 3.0 lbf, between about 2.5 lbf and bout 3.5 lbf, between about 3.0 lbf and about 4.0 lbf, between about 3.5 lbf and about 4.5 lbf, between about 4.0 lbf and about 5.0 lbf, between about 4.5 lbf and about 5.5 lbf, or between about 5.0 lbf and about 6.0 lbf, or otherwise, wherein 1 lbf corresponds to 4.448 newton) is applied to the contralateral guidewire.

Certain aspects of the present disclosure are directed toward a method of using the locking assembly described above. The method can include advancing a delivery system in a locked configuration. The deliver^{y} system can include a locking assembly fixed to the ipsilateral catheter. The locking assembly can include an interlock portion configured to retain the guidewire when the delivery system is in the locked configuration. A distal end of the guidewire can be introduced into the interlock portion from a distal side of the locking assembly such that the guidewire has a bend when the deliver^{y} system is in the locked configuration. The bend can be positioned between a proximal portion of the guidewire and the distal portion of the guidewire. The method can also include releasing the delivery system from the locked configuration to the unlocked configuration by advancing a release catheter along the guidewire.

### BRIEF DESCRIPTION OF THE DRAWINGS

These and other features, aspects and advantages will now be described in connection with certain embodiments, in reference to the accompanying drawings. The illustrated embodiments, however, are merely examples and are not intended to be limiting. The following are brief descriptions of the drawings.
FIG. 1A is a schematic representation of a bifurcated vascular prosthesis for use with the present disclosure, positioned at the bifurcation between the abdominal aorta and the right and left common iliac arteries.
FIG. 1B is an exploded view of a bifurcated graft for use with the present disclosure, showing a self-expanding wire support cage separated from an outer polymeric sleeve.
FIG. 2 is a schematic representation of an embodiment of the deployment catheter for delivering a bifurcated prosthesis, with a proximal portion of the main branch portion of the graft at least partially deployed.
FIG. 3 is a cross-sectional view of an embodiment of a deployment catheter for delivering a bifurcated prosthesis.
FIG. 4 is an enlargement of the portion delineated by curve 4-4 in FIG. 3.
FIG. 5 is a cross-sectional view of the embodiment of the deployment catheter shown in FIG.3 taken along line 5-5 of FIG. 4.
FIG. 6 is a cross-sectional view of the embodiment of the deployment catheter shown in FIG. 3 taken along line 6-6 of FIG. 4.
FIG. 7 is a schematic representation of an embodiment of the deployment catheter with the guidewire sheath positioned across the bifurcation.
FIG. 8 is a schematic representation, as in FIG. 7, with the deployment catheter positioned in the aorta.
FIG. 9 is a schematic representation, as in FIG. 8, with the compressed iliac branches of the graft positioned partly within the iliac arteries.
FIG. 10 is a schematic representation, as in FIG. 9, with a proximal portion of the main branch portion of the graft at least partially deployed within the aorta.
FIG. 11 is a schematic representation, as in FIG. 10, following the further proximal retraction of the guidewire sheath and the contralateral branch sheath through the contralateral iliac artery, causing the deployment of the contralateral branch portion of the graft.
FIG. 12A is a schematic representation, as in FIG. 11, following the proximal retraction of the ipsilateral branch sheath and deployment of the ipsilateral branch portion of the graft.
FIG. 12B is a schematic representation, as in FIG. 12A, following introduction of the release member at the contralateral access site and advancement of the release member along the contralateral guidewire.
FIG. 12C is a schematic representation, as in FIG. 12B, with a release member interfacing with a locking assembly.
FIG. 12D is a schematic representation, as in FIG. 12C, with a release member being retracted through the contralateral access site following decoupling of the contralateral guidewire from a locking assembly. In some embodiments, a cuff may be implanted (e.g., on a proximal end of the main branch portion) to secure or lengthen the graft.
FIG 13A is a schematic representation of a locking assembly configured to releasably retain an elongate member.
FIG. 13B is an isometric view of an exemplary embodiment of the locking assembly including a housing an elastomeric member.
FIG. 14 is an isometric view of an embodiment of the elastomeric member shown in FIG. 13B.
FIG. 14A is a top view of an embodiment of the elastomeric member shown in FIG. 14.
FIG. 14B is a rear view of an embodiment of the elastomeric member shown in FIG. 14.
FIG. 14C is a front cross-sectional view of an embodiment of the elastomeric member shown in FIG. 14.
FIG. 14D is a front view of an embodiment of the elastomeric member shown in FIG. 14.
FIG. 15 is a front cross-sectional view of an embodiment of the present locking assembly.
FIG. 16 is a front cross-sectional view of an alternative embodiment of the present locking assembly.
FIG. 17A is a front view of an embodiment of a locking assembly coupled to a contralateral guidewire.
FIG. 17B is a front view of the embodiment depicted in FIG. 17A interfacing with a release member.
FIG. 18A is an alternative embodiment of the locking assembly presently disclosed.
FIG. 18B is an alternative embodiment of the locking assembly presently disclosed.
FIG. 18C is an alternative embodiment of the locking assembly presently disclosed.
FIG. 18D is an alternative embodiment of the locking assembly presently disclosed.
FIG. 18E is an alternative embodiment of the locking assembly presently disclosed.
FIG. 18F is an alternative embodiment of the locking assembly presently disclosed.
FIG. 18G is an alternative embodiment of the locking assembly presently disclosed.
FIG. 18H is an alternative embodiment of the locking assembly presently disclosed.
FIG. 18I is an alternative embodiment of the locking assembly presently disclosed.
FIG. 18J is an alternative embodiment of the locking assembly presently disclosed.
FIG. 18K is an alternative embodiment of the locking assembly presently disclosed.
FIG. 18L is an alternative embodiment of the locking assembly presently disclosed.
FIG. 18M is an alternative embodiment of the locking assembly presently disclosed.
FIG. 18N is an alternative embodiment of the locking assembly presently disclosed.
FIG. 18O is an alternative embodiment of the locking assembly presently disclosed.
FIG. 18P is an alternative embodiment of the locking assembly presently disclosed.
FIG. 18Q is an alternative embodiment of the locking assembly presently disclosed.
FIG. 18R is an alternative embodiment of the locking assembly presently disclosed.
FIG. 19 is a side view of an embodiment of a contralateral guidewire.

### DETAILED DESCRIPTION

The following detailed description is now directed to certain specific embodiments of the disclosure. In this description, reference is made to the figures wherein like parts are designated with like numerals throughout the description and the drawings. Described below are various embodiments of a deliver^{y} system for establishing a surgical platform having multiple guidewires. In some aspects, the present disclosure is directed to devices and methods for deploying a vascular graft for treatment of an abdominal aortic aneurysm, including a deployment catheter and a guidewire assembly which may be used to maintain access through an implanted vascular graft for subsequent catheterizations.

An abdominal aortic aneurysm usually arises in the infrarenal portion of the diseased aorta, for example, below the kidneys. The aneurysm often occurs near a site of vessel branching, making a bifurcated stent a well-suited device for treating the abdominal aortic aneurysm. Endoluminal implantation is an increasingly accepted technique for implanting vascular grafts. This procedure may involve femoral cut down access or percutaneously inserting a vascular graft or prosthesis by using a delivery catheter. This process eliminates the need for major surgical intervention, thereby decreasing the risks associated with vascular and arterial surgery. Various embodiments of catheter delivery systems for prosthetic devices are described herein.

Endovascular surgical procedures can use a guidewire to help position a catheter or place prosthesis. Guidewires can provide a surgical platform from which a physician can conduct a minimally-invasive medical procedure. In some instances, a physician may use multiple guidewires in a medical procedure. When multiple guidewires are used, a first guidewire may have at least one of its ends in a location that is different from at least one of the ends of a second guidewire. When multiple guidewires are used, at least a portion of a first guidewire may be located in proximity to at least a portion of a second guidewire. In some instances, one end of a first guidewire may be located next to a portion of a second guidewire while the other end of the first guidewire is at a location different from the location of the end of the second guidewire. A first guidewire may access the patient at one location and be joined within the patient to a second guidewire that accesses the patient from a different location. Guidewires that access the patient's body from different locations can be used to deploy a bifurcated stent, to seat a heart valve, or to perform an endovascular surgical procedure.

Certain current delivery systems for a bifurcated stent graft system or a graft having at least one branch portion may use two separate sheaths to deploy the distal segment of the graft before the proximal segment. The outer sheath is first retracted to deploy a portion of the mid-body and the contralateral limb. Then, the front sheath is advanced distally to deploy the distal end of the graft. See e.g., U.S. Pat. No. 6,660,030. Other delivery systems, for example as disclosed in U.S. patent application Ser. No. 11/522,292, titled "A MULTI-SEGMENTED GRAFT DEPLOYMENT SYSTEM" and filed on Sep. 15, 2006 may use a plurality of axially spaced releasable restraint members temporarily connected by a pull wire to allow the distal main branch portion to be deployed before a proximal graft portion. Typically, these delivery systems are delivered to the aneurysm location over a guidewire. The guidewire may be further used to release a branch graft portion of the prosthesis, for example, by operably connecting a branch graft restraint mechanism to the guidewire and proximally withdrawing the guidewire from the vasculature.

Once the bifurcation graft has been deployed and implanted, a variety of procedures may desirably be accomplished. For example, it may be advantageous to implant a cuff (e.g,., on the proximal end of the main branch portion) to secure the graft and thereby prevent movement or slippage of the main branch portion. Alternatively, it may be necessary to dilate the stenosis or touch up or re-establish the expansion of the graft. These procedures require advancing another catheter to the graft location along a guidewire. However, the positioning of a guidewire through the graft after the graft has been deployed is difficult since the tip of the guidewire may snag on the wire support cage of the graft. Thus, it may be advantageous to provide a guidewire assembly configured to remain placed through a graft once the graft has been deployed and to allow access through the expanded graft for subsequent catheterizations. Additionally, it may be advantageous to improve the configuration of the deployment catheter and/or the graft restraining members so as to improve the methods of deploying and positioning bifurcated and non-bifurcated grafts, as will be described herein.

In certain embodiments, the deployment catheter may be configured to deliver a graft that includes a main or distal graft portion and at least one branch or proximal graft portion. In certain embodiments, the hollow guidewire assembly may be associated with a restraint member for the branch segment, such that the branch segment may be deployed by the guidewire assembly. The guidewire assembly may be further configured such that it may be used to remove the restraint member from the branch segment while permitting placement and maintenance of a guidewire through the expanded branch segment and main body graft for subsequent catheterizations. Other embodiments of a graft deployment system and guidewire assembly will also be described below.

### Prosthesis

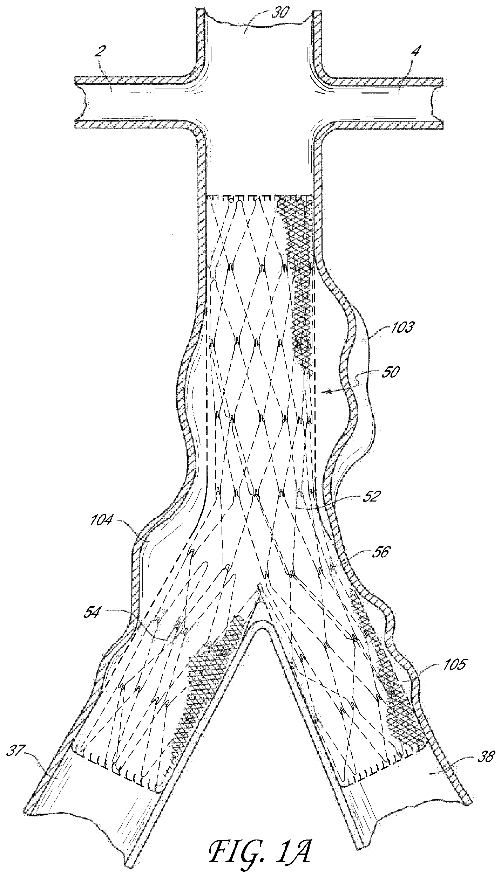

FIG. 1A is a schematic representation of an example of a bifurcated vascular graft **50** that can be used with any embodiment of the deployment catheter disclosed herein, positioned at the bifurcation between the abdominal aorta **30** and the right and left common iliac arteries **37** and **38.** With reference to FIG. 1A, there is illustrated a schematic representation of the abdominal part of the aorta and its principal branches. In particular, the abdominal aorta **30** is characterized by a right renal artery 2 and left renal artery **4.** The large terminal branches of the aorta **30** are the right and left common iliac arteries **37** and **38.** Additional vessels (e.g., second lumbar, testicular, inferior mesenteric, middle sacral) have been omitted from FIG. 1A for simplification. One embodiment of an expanded bifurcated endoluminal vascular prosthesis is shown spanning aneurysms **103, 104** and **105.** The expanded bifurcated endoluminal vascular graft **50** can comprise a main branch portion **52** (also referred to herein as a main branch segment) for traversing the aorta, a first branch portion **54** (also referred to herein as a first branch segment or an ipsilateral branch portion) for spanning an ipsilateral iliac artery **37**, and a second branch portion **56** (also referred to herein as a second branch segment or a contralateral branch portion) for spanning a contralateral iliac artery **38.**

The terms "first" and "second" may be used interchangeably. In one embodiment, the first branch portion can refer to a downstream or upstream portion of a main branch vessel. For example, in one embodiment, the main branch portion and the first branch portion are configured to lie within at least a portion aortic arch (including, for example, the ascending and/or descending aorta) with main branch portion positioned closer to the heart while the second branch portion can be configured to extend into one of the branch vessels (left subclavian, right subclavian or carotid) that extend from the aortic arch.

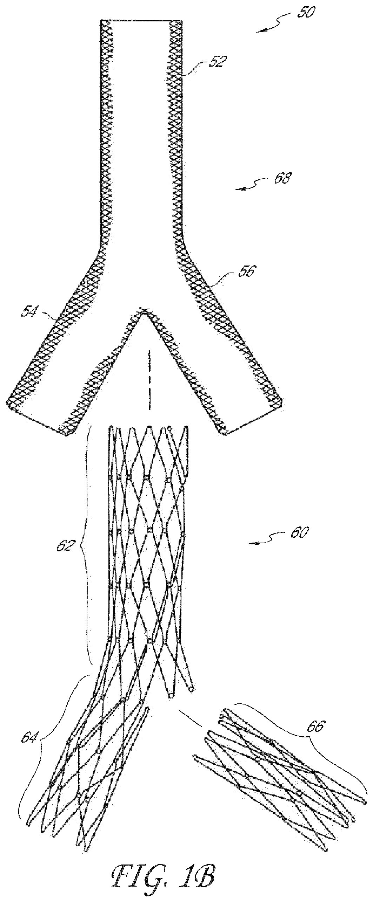

FIG. 1B is an exploded view of the bifurcated graft **50** of FIG. 1A, which can include a self-expanding wire support cage **60** and an outer polymeric sleeve **68.** In FIG. 1B, the wire support **60** is shown separated from an outer polymeric sleeve **68.** In the illustrated embodiment, the polymeric sleeve **68** can be situated concentrically outside of the tubular wire support **60.** However, other embodiments may include a sleeve positioned instead concentrically inside the wire support or positioned on both the inside and the outside of the wire support. Alternatively, the wire support may be embedded within a polymeric matrix or layer which makes up the sleeve. The sleeve **68** may be attached to the wire support 60 by any of a variety of suitable manners known to those skilled in the art.

The tubular wire support **60** can comprise a main branch portion **62** for traversing the aorta, a first branch portion **64** (also referred to herein as an ipsilateral branch portion) for spanning an ipsilateral iliac and a second branch portion **66** (also referred to herein as a contralateral branch portion) for spanning a contralateral iliac. The main branch portion **62** and first ipsilateral branch portion **64** can be formed from a continuous single length of wire having a proximal end, a distal end and a central lumen extending therebetween. Alternatively, the first ipsilateral branch portion **64** may be formed of one or more lengths of wire pivotably connected to the proximal end of the main branch portion **62.** A second, contralateral branch component **66** may be formed of one or more lengths of wire pivotably connected to the proximal end of the main branch portion **62.** Each of the iliac branch components has a proximal end, a distal end and a central lumen extending therethrough. Construction of the graft from a three part cage conveniently facilitates the use of different gauge wire in the different components (e.g. 0.014 in. diameter main trunk and 0.012 in. diameter branch components).

In general, each of the components of the bifurcated endoluminal vascular graft **50** may vary considerably in diameter, length, expansion coefficient, and other parameters or characteristics, depending upon the intended application. For implantation within the aorta of a typical adult, the main branch portion **52** will have a length within the range of from approximately 2 in. or less to approximately 5 in. or more, and, typically within the range of from approximately 3.5 in. to approximately 4 in. The unconstrained outside expanded diameter of the main branch portion **52** will typically be within the range of from approximately 0.75 in. to approximately 1.5 in. The unconstrained expanded outside diameter of the main branch portion **52** can be constant or substantially constant throughout the length, or can be tapered from a relatively larger diameter at the distal end to a relatively smaller diameter at the bifurcation. In general, the diameter of the proximal end of the main branch portion will be on the order of no more than approximately 95% and often no more than approximately 85% of the diameter of the distal end of the main branch portion. The iliac branch portions **54** and **56** will typically be bilaterally symmetrical, having a length within the range of from approximately 0.4 in. to approximately 2.6 in., and a diameter within the range of from approximately 0.04 in. to approximately 0.79 in.

The collapsed prosthesis for use in accordance with the present disclosure has a diameter in the range of approximately 0.08 in. to approximately 0.39 in. The maximum diameter of the collapsed prosthesis can be in the range of approximately 0.12 in. to approximately 0.24 in. (12 to 18 French). Some embodiments of the deployment catheter, including the prosthesis, can have a diameter in the range of from approximately 18 to approximately 20 or approximately 21 French. Other embodiments can have a diameter as low as approximately 19 French, approximately 16 French, approximately 14 French, or smaller. After deployment, the expanded endoluminal vascular prosthesis may radially self-expand to a diameter anywhere in the range of approximately 0.8 in. to approximately 1.6 in.

Although certain prosthesis configurations are disclosed herein, these are only examples of prostheses which are deployable using the embodiments of a deployment catheter and guidewire assembly described herein. In other embodiments, the delivery system described below may be used to deliver and deploy other types of self-expandable bifurcated or multi-segmented prosthesis having a main branch portion and at least one branch graft portion, as will be apparent to those of skill in the art in view of the disclosure herein. For example, in other embodiments, certain features and aspects of the deployment catheter and guidewire assembly can be used to deploy a graft without a branch graft portion, a graft with only one branch portion and/or a graft with more than one graft portions. Further details and additional embodiments of the prosthesis described above can be found in U.S. Pat. Nos. 6,007,296, 6,187,036, and 6,197,049.

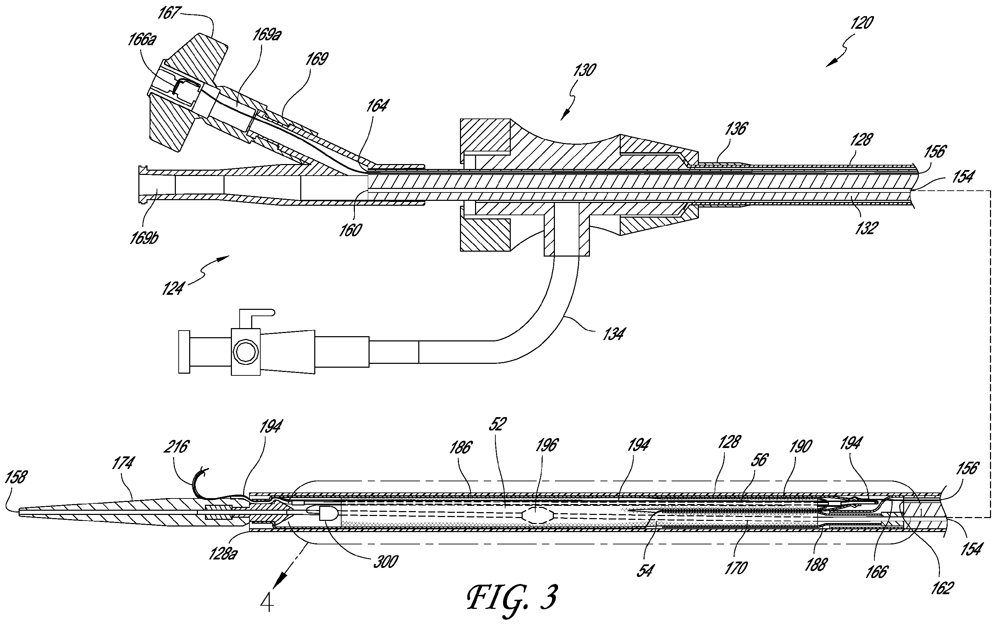

It should also be appreciated that, although the illustrated embodiments are described in the context of a bifurcated graft configured for the abdominal aorta, certain features and aspects of the delivery systems and methods described herein can be used in other portions of the vascular system. For example, it is anticipated that certain features and aspects of the systems and methods described herein can be adapted for use in the thoracic aorta. In some embodiments, the deployment catheter **120** (see FIG. 3) may be configured to treat defects that may include, but are not limited to, abdominal aortic aneurysms, subclavian aneurysms, and thoracic aortic aneurysms, to name a few. It is also anticipated that certain
features and aspects of the system described herein may be adapted to deliver a single straight graft segment to the thoracic aorta or other vessels or arteries within the body.

### Delivery System

The expandable bifurcation graft **50** can be deployed at a treatment site with any of a variety of deployment catheters, as will be apparent to those of skill in the art. Any of the embodiments of the deployment catheters disclosed herein may comprise any of the materials, features, or other details of any deployment catheters suitable for deploying an expandable bifurcation graft known in the field. Further details and additional embodiments of the deployment catheter can be found in U.S. Pat. Nos. 8,236,040 and 8,523,931.

The deployment catheters herein disclosed can be used for deploying a self-expanding bifurcation graft known in the field, or in any of the embodiments disclosed in U.S. Pat. No. 6,090,128, U.S. Pat. No. 6,500,202, U.S. Pat. No. 6,660,030, U.S. Pat. No. 8,523,931, and U.S. Pat. Pub. 2008/0071343.

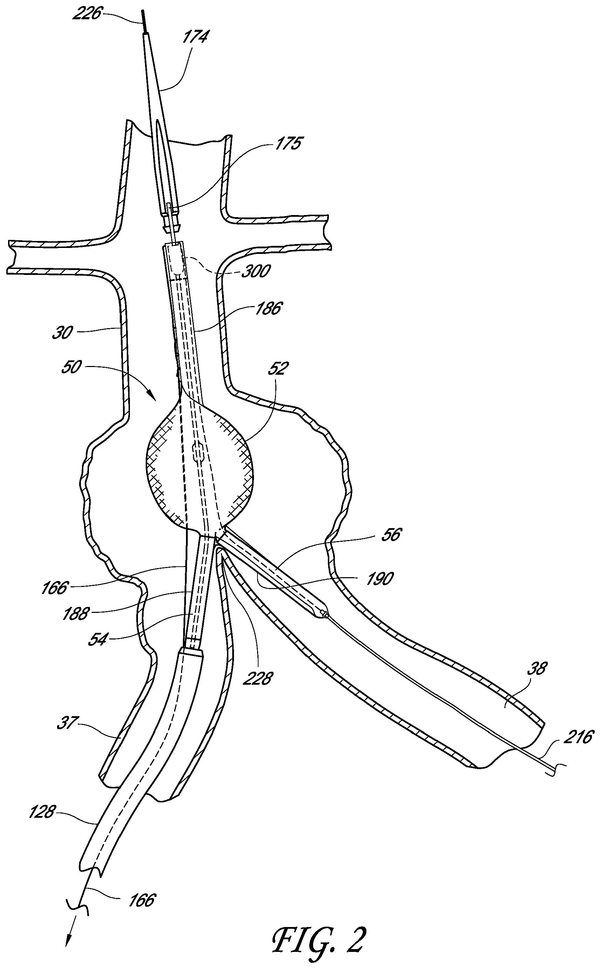

With reference to FIG. 2, one method for using an embodiment of a deployment catheter **120** for treating an abdominal aortic aneurysm will be briefly described, without limitation. More detail regarding this deployment method will be described below.

FIG. 2 is a schematic representation of an embodiment of a deployment catheter **120** for delivering a bifurcated prosthesis or graft **50,** showing a proximal portion of the main branch portion **52** of the graft **50** at least partially deployed within the aorta for illustration purposes. As shown in FIG. 2, the deployment catheter **120** can be introduced into a patient's vasculature through a puncture site in the patient's ipsilateral artery. The deployment catheter **120** is not limited to treatment of an abdominal aortic aneurysm; it can be configured to treat other aneurysms as discussed more fully herein. Additionally, depending on the clinical requirements, the deployment catheter **120** can be introduced into the patient's vasculature through puncture sites other than an ipsilateral artery. For example, without limitation, the deployment catheter **120** can be introduced into the patient's vasculature through a contralateral artery, through a radial artery, or through a subclavian artery.

As illustrated in FIG. 2, the deployment catheter 120 can be advanced over a guidewire **226** to the desired location within the patient's aorta. The graft 50 illustrated in FIG. 2 can include a main branch portion **52** constrained within a main branch sheath or member **186**, an ipsilateral branch portion **54** constrained within and ipsilateral branch sheath or member **188,** and a contralateral branch portion **56** constrained within a contralateral branch sheath or member **190.** Prior to the deployment of the main branch portion **52** of the graft **50** as shown in FIG. 2, the entire graft can be constrained within an outer sheath **128** of the deployment catheter **120.** In brief, the graft **50** can be exposed by retracting the outer sheath **128,** and the deployment catheter **120** can be manipulated so as to position the contralateral branch portion **56** in the contralateral artery **38.**

After positioning the graft **50** in the desired position, illustrated in FIG. 2, the main branch portion **52** of the graft **50** can be deployed by retracting a sheath release **166** (e.g., a cord, suture, wire, or likewise), which can cause the perforated main branch sheath **186** to tear along a side thereof. The remaining portion of the main branch portion **52** can be deployed by further withdrawing the sheath release **166.** The main branch sheath **186** can be attached to the sheath release **166,** allowing the main branch sheath **186** to be removed through the ipsilateral access site as the sheath release **166** is removed through the ipsilateral access site. In other configurations, the main branch sheath **186** can be separately withdrawn from the contralateral access site or with either the ipsilateral branch sheath **188** or the contralateral branch sheath **190.**

In the illustrated embodiment, the contralateral branch portion **56** of the graft 50 can be deployed by withdrawing a contralateral guidewire sheath **216** through a puncture site in the contralateral iliac artery **38,** causing the contralateral branch sheath **190** to be withdrawn. Similarly, the ipsilateral branch portion **54** of the graft **50** can be deployed by withdrawing the deployment catheter **120** through a puncture site in the ipsilateral iliac artery **37,** causing the ipsilateral branch sheath **188** to be withdrawn either before or after the contralateral branch sheath **190** is withdrawn.

The deployment method described with reference to FIG. 2 is not intended to limit the applicability of the deployment catheter **120.** The deployment catheter **120** may be configured to deploy a straight, bifurcated, or any other graft configuration into any portion of an artery or other blood vessel in the body. In some embodiments, the deployment catheter 120 may be used to deploy grafts having anchoring elements that help secure the graft to the vessel wall as well as grafts that do not have anchoring elements. With this brief, non-limiting overview of one method of using the deployment catheter **120** having been described, additional features and configurations of the deployment catheter **120** and additional details of this and other deployment methods will now be described.

FIG. 3 is a cut-away side view of a non-limiting exemplary embodiment of a deployment catheter **120.** The inner core **132** of the deployment catheter **120** can include a guidewire lumen **154** and a sheath release lumen **156** extending longitudinally therethrough. The guidewire lumen **154** can be defined by a central tube **170** that can be disposed within inner core **132.** The guidewire lumen **154** can be defined by a hole bored along a longitudinal axis of the inner core **132.** In the illustrated embodiment, the guidewire lumen **154** can extend throughout the entire length of the tubular inner core **132,** having a distal exit port **158** and a proximal access port **160,** as will be understood by those of skill in the art. In use, the deployment catheter **120** can be advanced into position in the aorta over a guidewire **226** (shown in FIG. 2) extending through the guidewire lumen **154,** as will be understood by those of skill in the art. A sheath release **166** (also may be referred to herein as a cord) can be routed through the sheath release lumen **156.** In the illustrated embodiment, the sheath release lumen **156** can extend through the entire length of the tubular inner core **132,** having a distal exit port **162** and a proximal access port **164,** as will be understood by those of skill in the art.

In the embodiment of the deployment catheter **120,** the guidewire lumen **154** can be co-planar with the centerline axis of the inner core **132** and the sheath release lumen **156.** However, this arrangement is not required. In some embodiments, the guidewire lumen **154** can be not coplanar with the centerline axis of the inner core **132** and the sheath release lumen **156.** Therefore, the inner core **132** may be configured so that the guidewire lumen **154** and the sheath release lumen **156** are formed at any desired position in the cross-section of the inner core **132.**

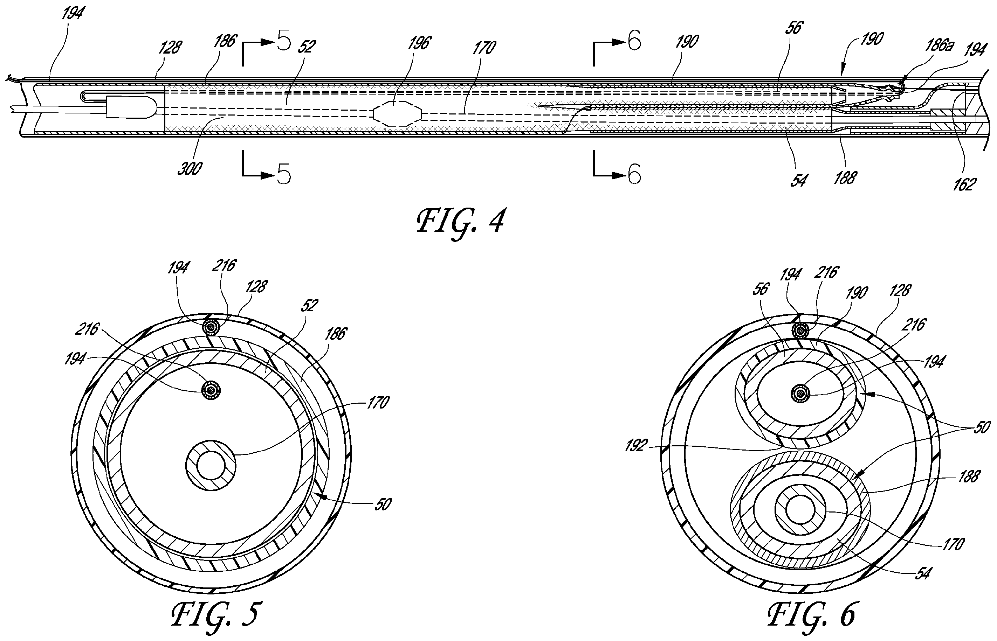

FIG. 4 is an enlargement of the portion delineated by the curve **4** in FIG. 3. FIGS. 5 and 6 are a cross-sectional view of the embodiment of the deployment catheter 120 shown in FIG. 3 taken along line **5-5** and line **6-6,** respectively, of FIG. 4. With reference to FIGS. 4-6, a bifurcated endoluminal graft **50** is illustrated in a compressed configuration within the deployment catheter **120,** prior to the advancement of the inner core **132** relative to the other sheath **128.** The graft **50** can comprise a distal aortic trunk or main branch portion **52,** a proximal ipsilateral branch portion **54,** and a proximal contralateral iliac portion **56.** In the illustrated embodiment, the aortic main branch portion **52** of the graft **50** can be constrained within a main branch sheath **186.** While the embodiment of main branch sheath **186** is shown with reference to compressing a main branch graft portion **52,** it is envisioned that the sheath **186** could alternatively be used to compress and deliver other portions of a multi-segmented vascular graft, such as a branch graft portion, the entire multi-segmented graft, or a single-segment, straight vascular graft. Further, in the illustrated embodiment, the ipsilateral branch portion **54** can be constrained with a tubular ipsilateral branch sheath **188** (also referred to herein as the first branch sheath), and the contralateral branch portion **56** (also referred to herein as the second branch sheath) can be constrained within a generally tubular contralateral branch sheath **190.** In the illustrated embodiment, the ipsilateral branch sheath **188** and the contralateral branch sheath **190** can be open-ended tubular sheaths.

The ipsilateral branch sheath **188** can constrain substantially the entire length of the ipsilateral branch portion **54** of the bifurcated graft **50.** Similarly, in the illustrated embodiment, the contralateral branch sheath **190** can constrain substantially the entire length of the contralateral branch portion **56** of the bifurcated graft **50.** However, in some embodiments, the ipsilateral branch sheath **188** and/or the contralateral branch sheath **190** may constrain substantially more or less than the entire length of the ipsilateral branch portion **54** or the contralateral branch portion **56,** respectively, of the bifurcated graft **50.**

With reference to FIG. 5, the main branch sheath **186** can be sized and configured to circumferentially surround the main branch portion **52** of the bifurcated graft **50.** However, in some embodiments, the main branch sheath **186** can be configured to only partially surround the main branch portion **52** of the bifurcated graft **50.** The main branch sheath **186** may extend to the distal end of the contralateral branch portion **56** of the graft **50.** In some embodiments, the main branch sheath **186** can be configured so as to define a notch 192 along the portion of the length of the main branch sheath **186** that covers the contralateral branch portion **56.** In some embodiments, the notch **192** can be a slit along a portion of the length of the main branch sheath **186.** In some embodiments, as in the illustrated embodiment, the notch **192** can remove a portion of the main branch sheath **186** along a portion of the length of the main branch sheath **186** that can be less than or equal to approximately half of the perimeter of the main branch sheath **186.** In some embodiments, the main branch sheath **186** can be skived to remove a suitable amount of the material comprising the main branch sheath **186** to allow the ipsilateral or contralateral branch portion **54, 56** of the graft **50** to deploy upon retraction of the outer sheath **128.** Thus, in some embodiments, the main branch sheath **186** may not constrain the ipsilateral or contralateral branch portion **54, 56** of the bifurcated endoluminal graft **50.**

In some embodiments, as illustrated in FIG. 4, a torsion tab **196** can be integrally formed with the central tube **170**, or secured thereto such as by thermal bonding, adhesive bonding, and/or any of a variety of other securing techniques known in the art. As is illustrated, the main branch portion **52** of the bifurcated endoluminal graft **50** can be constrained by the main branch sheath **186** around the torsion tab **196.** In the illustrated embodiment, the torsion tab **196** can engage with the endoskeleton or, with reference to FIG. 1B, the wire support cage **60** of the bifurcated graft **50** and ensures that the bifurcated graft **50** substantially rotates with the inner core **132** of the deployment catheter **120.** In other words, the torsion tab **196** can prevent the central tube **170** from rotating relative to the bifurcated graft **50.** This can enhance the ability of the medical practitioner or user to rotate and, hence, maneuver, the graft **50** and the ipsilateral and/or contralateral branch portions **54, 56** within the patient's aorta by rotating the proximal end of the deployment catheter **120,** in particular, by rotating the proximal end of the inner core **132** or the "Y" connector **169.** As such, the torsion tab **196** can cause the bifurcated endoluminal graft **50** to rotate substantially in unison with the central tube **170.**

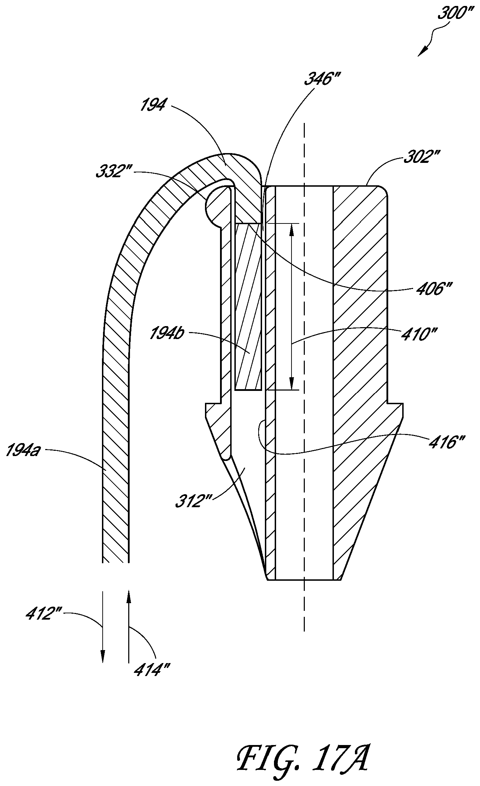

As described in greater detail below, a locking assembly **300** can couple with the central tube **170**. The locking assembly **300** may be integrally formed with the central tube **170,** or secured thereto such as by thermal bonding, adhesive bonding, and/or any of a variety of other securing techniques known in the art. The locking assembly **300** can engage a locking portion **194b** (shown in FIG. 17A) of a contralateral guidewire **194.** The locking portion **194b** is also referred to herein as a distal end **194b** or as a stiff region. The contralateral guidewire **194** can extend distally from the locking assembly **300** and cannulate the guidewire sheath **216.** The contralateral guidewire **194** can then bend proximally back and extend through the main branch portion **52** of the graft **50** and into the contralateral branch portion **56** of the graft **50.** The contralateral guidewire **194** and the contralateral guidewire sheath **216** can extend proximally from the contralateral branch portion **56** of the graft **50** and bend back distally, running distally along a gap formed between an inner surface of the outer sheath **128** and an outer surface of the main branch sheath 186. The contralateral guidewire **194** and contralateral guidewire sheath **216** can then exit the deliver^{y} catheter **120** through a gap formed between a proximal face of the distal tip **174** and a distal face of the outer sheath **128.** The distal tip **174** may include a groove (not shown) that accommodates the contralateral wire **194** as the contralateral wire **194** passes through the junction between the distal tip **174** and the outer sheath **128**.

The contralateral branch sheath **190** can be deployed using the contralateral guidewire sheath **216.** The ipsilateral branch sheath **188** can be connected to the inner core **132** or the interface member **168** and adapted to be axially proximally withdrawn from the ipsilateral branch portion **182** of the graft **178,** thereby permitting the ipsilateral branch portion **182** to expand to its implanted configuration. The main branch sheath 186 can retracted with the contralateral branch sheath **190** or with the ipsilateral branch sheath **188.**

### Method of Use

With reference to the embodiments of the deployment catheter **120** described above, an exemplary procedure or method of using the deployment catheter **120** to treat a patient's abdominal aortic aneurysm using the embodiments of the bifurcated endoluminal graft **50** disclosed above will now be described. However, the methods and devices of the present disclosure are not to be taken as limited to this particular illustrative example. The present methods and systems can be used in any medical procedure where one desires to reversibly couple catheters, sheaths, guidewires, or similar devices.

In the illustrated embodiment the main branch sheath **186** and the ipsilateral branch sheath **188** are introduced into the patient through the ipsilateral access site and removed from the patient through the ipsilateral access site, while the contralateral branch sheath 190 is introduced through the ipsilateral access site and removed through the contralateral access site.

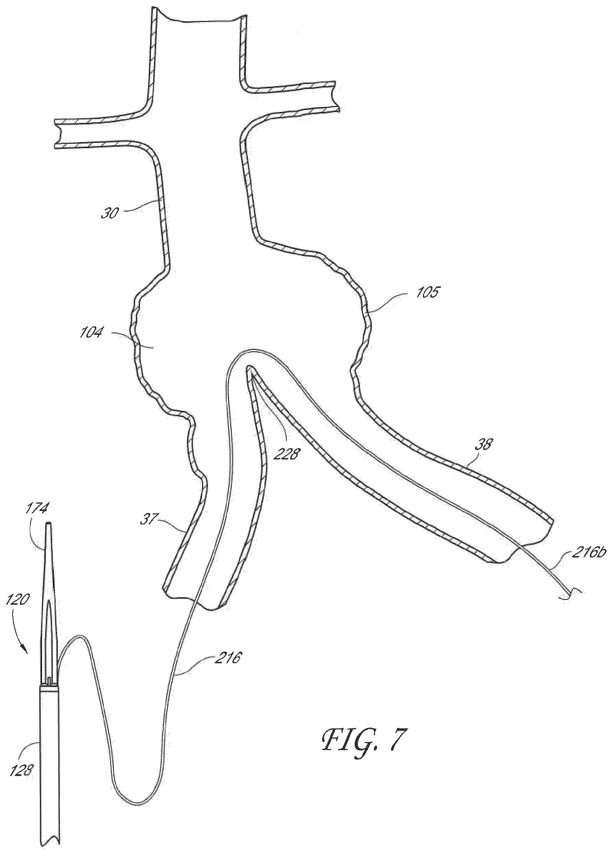

FIG. 7 is a schematic representation of an embodiment of the deployment catheter **120** with the contralateral guidewire sheath **216** positioned across the bifurcation and within the contralateral iliac artery **38.** The hollow contralateral guidewire sheath **216** can be introduced into the ipsilateral iliac artery **37** through an ipsilateral access site in the femoral artery, advanced superiorly towards the aorta **30,** and using cross-over techniques known to those skilled in the arts, subsequently advanced inferiorly down the contralateral iliac artery **38** and out a contralateral access site in the contralateral femoral artery. The leading portion **216 b** of the contralateral guidewire sheath **216** can be externalized by passing the leading portion **216 b** of the contralateral guidewire sheath **216** through the contralateral access site. As discussed below, the guidewire sheath **216** can be secured to the contralateral branch sheath **190.** The contralateral branch portion **56** of the bifurcated graft **50** can be deployed by withdrawing the contralateral guidewire sheath **216** and thereby removing the contralateral branch sheath **190** from the contralateral branch portion **56** of the graft **50**. The contralateral branch sheath **190** can be removed through the contralateral access site by pulling on the contralateral guidewire sheath **216.**

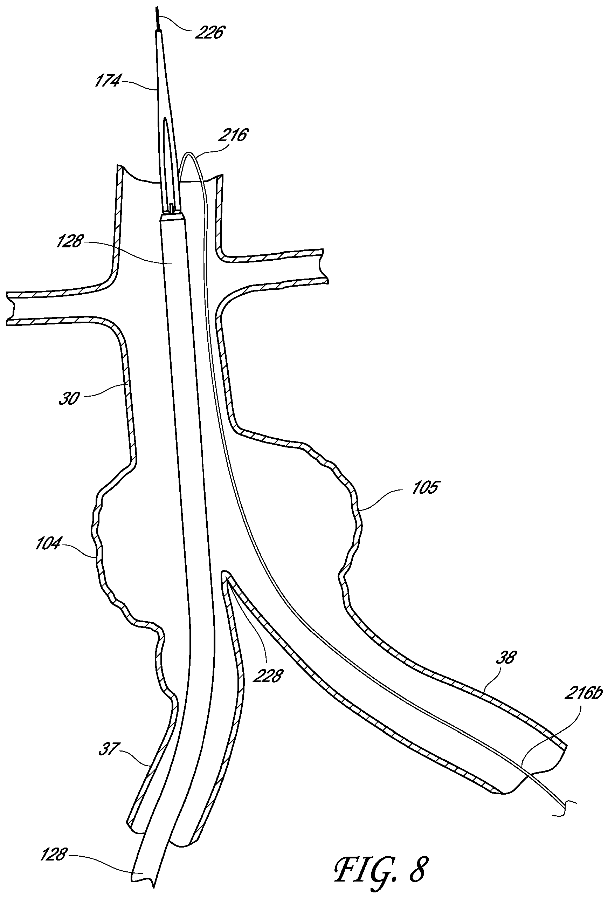

FIG. 8 is a schematic representation, as in FIG. 7, with the deployment catheter **120** positioned in the aorta **30.** Referring to FIG. 8, after the contralateral guidewire sheath **216** the has been positioned across the bifurcation **228** in the aorta **30,** the deployment catheter **120** can then be advanced over a second guidewire **226** (also referred to as the main guidewire), such as but not limited to a standard 0.035 in. guidewire, from the ipsilateral access site into the aorta **30** using techniques known to those skilled in the arts. Traction can be applied to the hollow contralateral guidewire sheath **216** from the contralateral access site to take up the slack in the contralateral guidewire sheath **216** as the deployment catheter **120** is advanced into the aorta **30.**

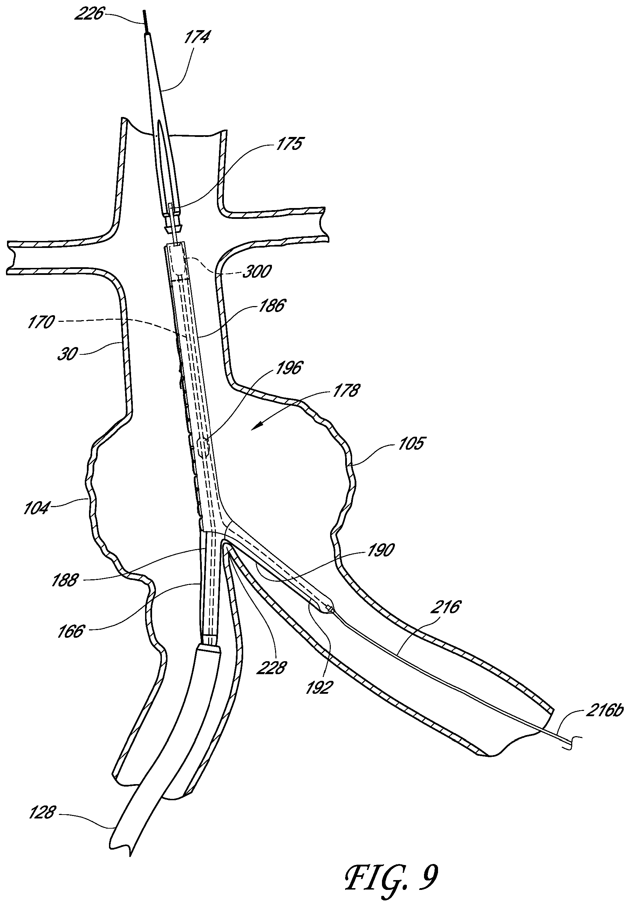

FIG. 9 is a schematic representation with the ipsilateral and contralateral branch portions **54, 56** of the graft **50** compressed within the ipsilateral and contralateral branch sheaths **188, 190** (respectively) and positioned substantially fully within the respective ipsilateral and contralateral iliac arteries. As shown in FIG. 9, the bifurcated graft **50** can be configured so as to abut against the bifurcation of the aorta **228** or be positioned in the vicinity of the bifurcation of the aorta **228** by retracting the deployment catheter **120** and, if desired, the contralateral guidewire sheath **216** until the bifurcated graft **50** abuts or is in the vicinity of bifurcation of the aorta **228.** The contralateral guidewire **194** can be manipulated so as to seat the graft **50** onto the bifurcation **228** of the aorta **30.**

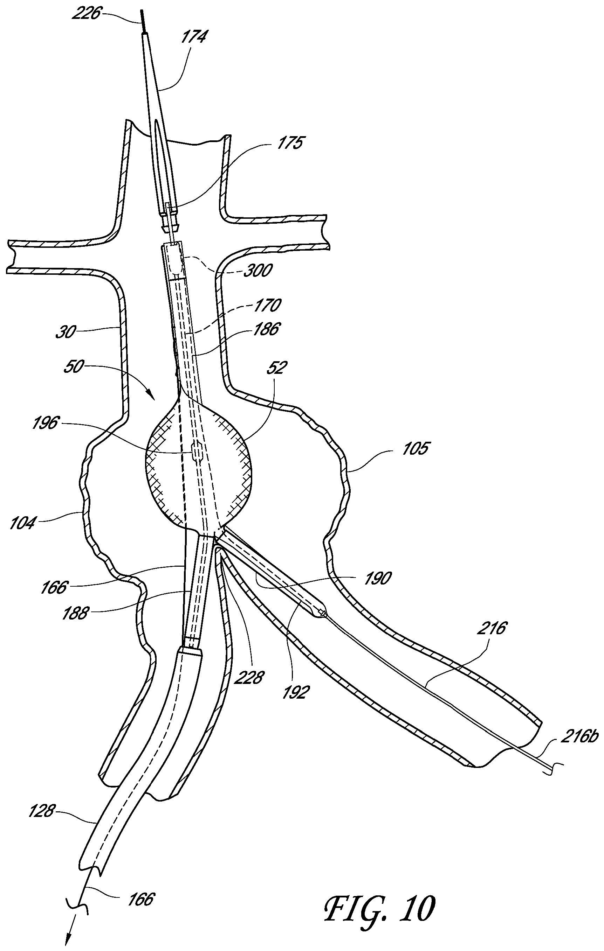

FIG. 10 is a schematic representation, as in FIG. 9, with a proximal portion of the main branch portion **52** of the graft **50** or at least partially deployed within the aorta **30.** The proximal portion of the main branch portion **52** of the graft **50** can be partially deployed within the aorta **30** as illustrated by proximally retracting the sheath release wire 166, as described above, while holding the inner core **132** of the deployment catheter (see FIG. 3) in a fixed position relative to the aorta **30** so as to prevent exerting undue force on the bifurcation **228** of the aorta **30** or other portions of the anatomy. Deploying the graft **50** in a bottom up sequence, as illustrated herein, may help mitigate the "wind socking" effect that can cause proximal migration of the graft **50.** Additionally, deploying the graft **50** and a bottom up sequence may allow for either axially or rotationally repositioning of a partially deployed graft **50** without causing significant or any damage to the arterial wall. In some embodiments, this may partly be due to the fact that the deployed middle portion of the graft **50** may move against the arterial wall more easily than a deployed end portion of the graft **50.** The main branch sheath **186** can be attached to the sheath release wire **166** and withdrawn from the patient through the ipsilateral access site.

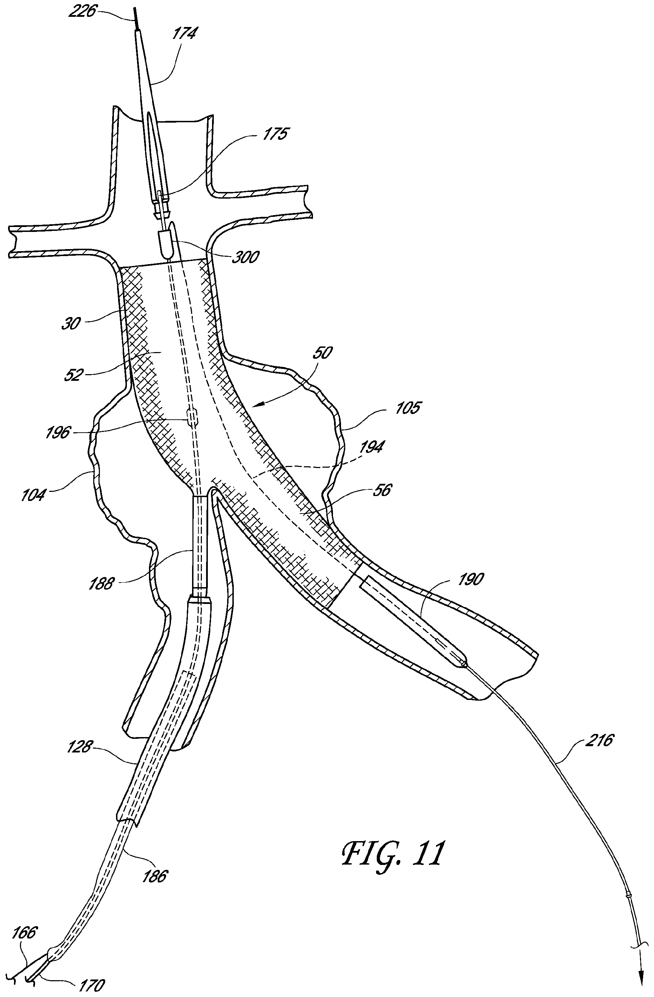

FIG. 11 is a schematic representation, as in FIG. 10, following the further proximal retraction of the contralateral guidewire sheath **216** and, consequently, the contralateral branch sheath **190,** through the contralateral iliac artery **38.** As illustrated therein, the contralateral branch sheath **190** has been retracted so as to completely deploy the contralateral branch portion **56** of the bifurcated graft **50.** The contralateral guidewire **194** may remain coupled to the locking assembly **300** as the contralateral guidewire sheath **216** is withdrawn through the contralateral access site.

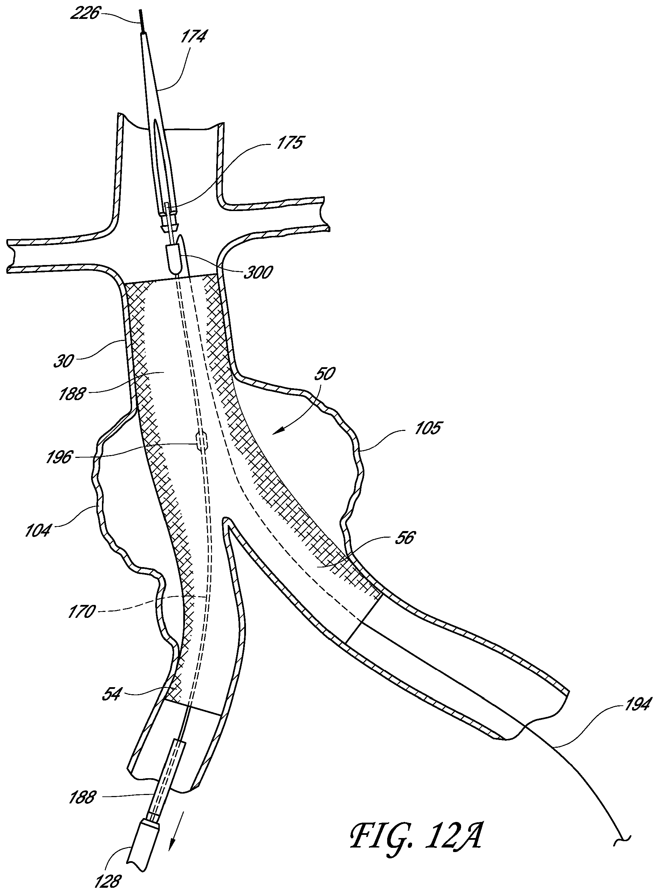

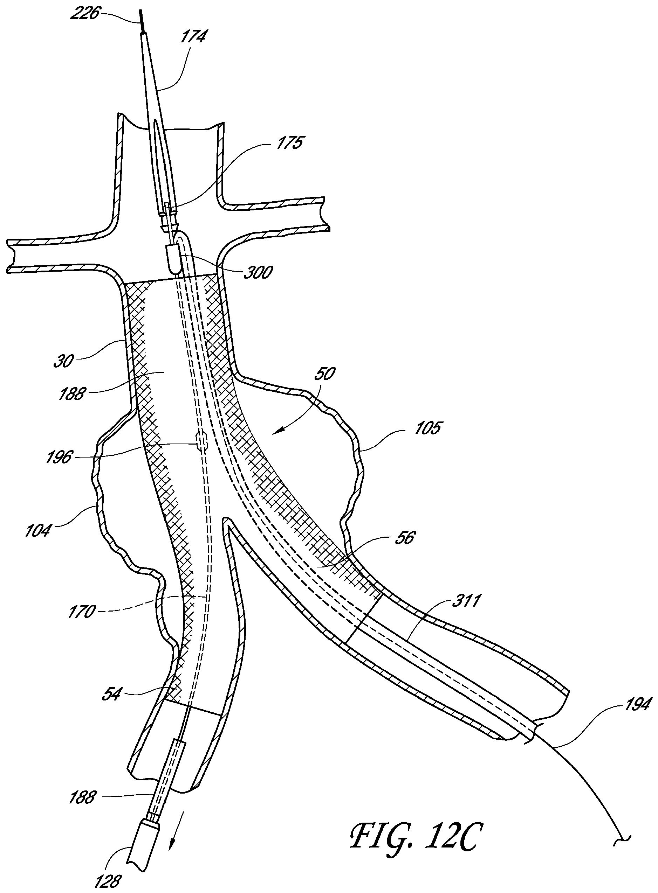

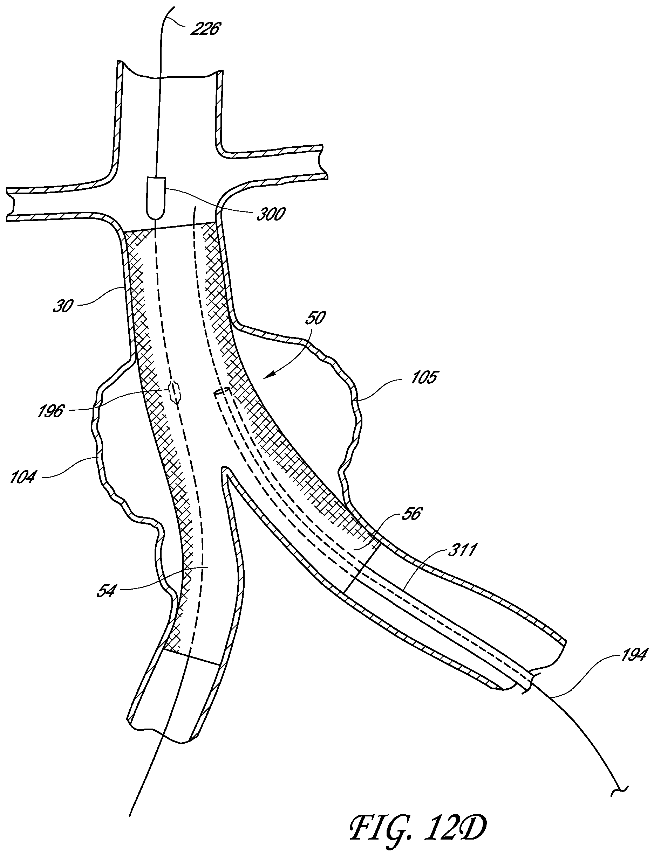

FIG. 12A is a schematic representation, as in FIG. 11, following the proximal retraction of the ipsilateral branch sheath **188** and deployment of the ipsilateral branch portion **54** of the graft **50.** The ipsilateral branch portion **54** of the graft **50** may be deployed by proximally retracting the inner core **132** which, as described above, can be directly or indirectly rigidly attached to the ipsilateral branch sheath **188** (see FIG. 3). Because the ipsilateral branch sheath **188** can be an open-ended tubular sheath, the ipsilateral branch portion **54** of the graft **50** can be deployed in a top down sequence.

However, the ipsilateral branch sheath **188** (and the contralateral branch sheath **190)** can be configured to accommodate any other desired or suitable sequence. In some embodiments, the ipsilateral branch portion **54** of the bifurcated graft **50** may be deployed before deployment of the contralateral branch portion **56** of the graft **50.** Additionally, although the figures illustrate the main branch portion **52** of the graft **50** being deployed with the contralateral branch portion **56,** in other embodiments, the main branch portion **52** of the graft **50** may be deployed with the ipsilateral branch portion 54. Also, although the figures illustrate the ipsilateral branch portion 54 being deployed before the contralateral branch portion 56, in other methods, the contralateral branch portion 56 may be deployed before the ipsilateral branch portion 54 of the graft 50.

In the illustrated embodiment depicted in FIG. 12A, the contralateral guidewire **194** remains coupled to the locking assembly **300** after deployment of the bifurcated graft **50.** The locking assembly **300** retains the distal end **194b** (shown in FIG. 17A) of the contralateral guidewire **194** to prevent unintended movement of the distal end of the contralateral guidewire **194.** The locking assembly **300** can be configured to allow the medical technician to actuate the locking assembly **300,** thereby triggering the locking assembly **300** to release the distal end of the contralateral guidewire **194.**

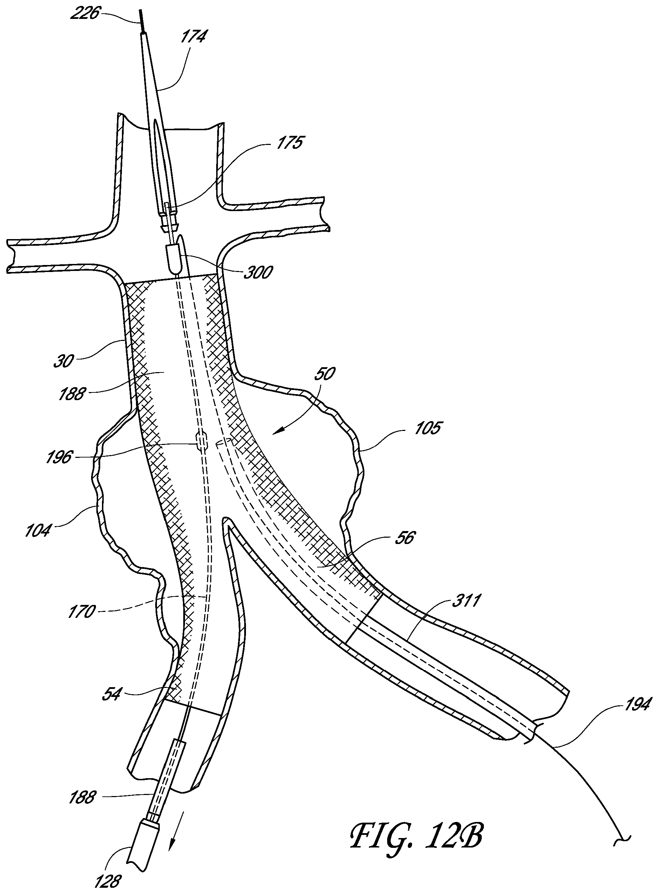

Referring to FIGS. 12B-D, release of the contralateral guidewire **194** from the locking assembly **300** may be accomplished by advancing a release member **311** (e.g., pigtail catheter) along the contralateral guidewire **194.** The release member **311** can then directly or indirectly apply a vertical force to the contralateral guidewire **194,** thereby causing the locking assembly **300** to release the distal end **194b** of the contralateral guidewire **194.** The release member **311** can then be withdrawn through the contralateral access site. The contralateral guidewire **194** can be withdrawn before, after, or at the same time as the withdrawal of the release member **311.** Additionally or alternatively, the release member **311** can be configured to release the contralateral guidewire **194** upon the activation of a triggering element that is coupled to the locking device **300,** as described below.

Although the illustrated method shows the ipsilateral branch portion **54** of the graft **50** being released before withdrawing the contralateral guidewire **194,** in some methods, the contralateral guidewire **194** may be released (e.g., as shown in FIGS. 12B-12D) prior to releasing the ipsilateral branch portion **54** (e.g., as shown in FIG. 12A).

### Locking Assembly

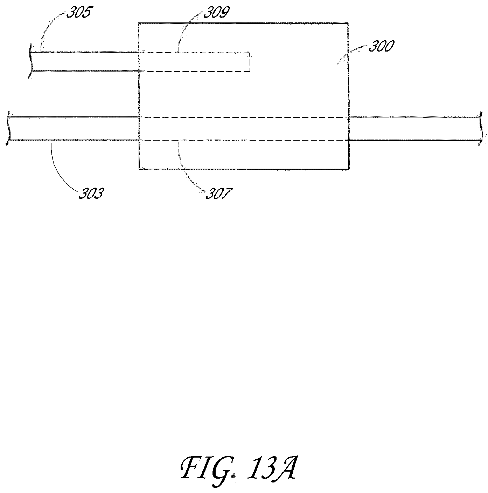

FIG. 13A is a schematic representation of a locking assembly **300.** The locking assembly **300** may be interfaced with (e.g., removably coupled, permanently secured, or integrally formed with) a first elongate member **303** and a second elongate member **305.** The locking assembly **300** may be configured to interface with more than two elongate members. The first elongate member **303** may pass through the locking assembly **300.** The locking assembly **300** can interface with an anchor portion **307** of the first elongate member **303.** The locking assembly **300** may reversibly couple to a locking portion **309** of the second elongate member **305.**

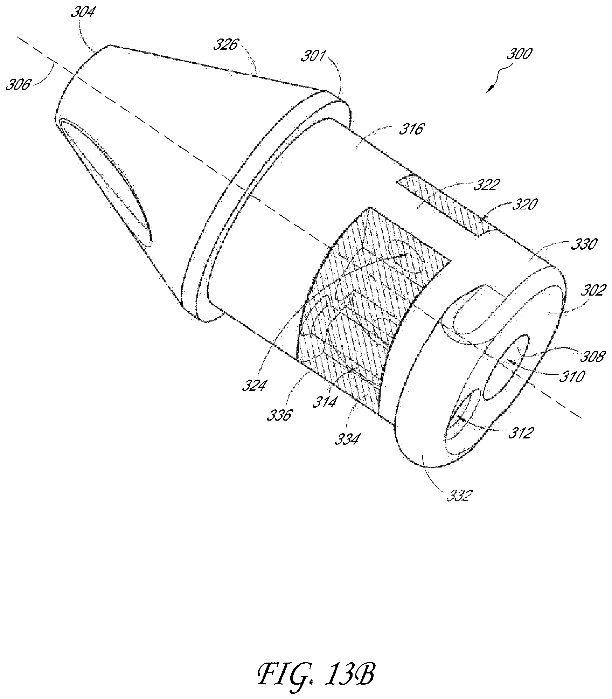

FIG. 13B is a non-limiting example of a locking assembly **300** that can be used with any embodiment of the deployment catheter **120** disclosed herein. In general, the locking assembly **300** presently disclosed can be configured to secure two elongate members to the locking assembly **300.** The locking assembly **300** can be adapted to release one of the elongate members from the locking assembly **300** when a user applies a vertical force to the elongate member being released. The locking assembly **300** presently disclosed can be used in medical procedures that employ a junction of two wires from different locations, such as procedures deploying bifurcated stents or multiple stents in thoracic, renal, or cardiac procedures. By way of a non-limiting example, the locking assembly **300** can be used to join a contralateral guidewire to an ipsilateral catheter when deploying a bifurcated stent in order to treat an abdominal aortic aneurysm. Certain aspects of the locking assembly **300** will now be described by presenting a variety of non-limiting exemplary embodiments of the locking assembly **300.**

Referring to FIG. 13B, the locking assembly **300** can include a housing **301** having a distal face **302** and a proximal face **304.** As shown in FIG. 13B, the proximal and distal faces **304, 302** may be substantially perpendicular to the longitudinal axis **306.** However, in other configurations the proximal and distal faces **304, 302** may not be substantially perpendicular to the longitudinal axis **306** and/or parallel with each other. The locking assembly **300** can include an anchoring surface **308** that defines a first lumen **310.** The first lumen **310** may communicate between the distal face **302** and the proximal face **304** of the housing **301.** As shown in FIG. 13B, the first lumen **310** may be concentric to the longitudinal axis **306** of the locking assembly **300.** However in other configurations, the first lumen **310** may be off-center of the longitudinal axis **306** of the locking assembly **300.**

The elongate member can pass through the first lumen **310** of the locking assembly **300**, e.g., the central tube **170** of the delivery catheter **120** can pass through the first lumen **310.** The locking assembly **300** can be integral with the central tube **170** or bonded to the central tube **170** so that there is no relative movement between the locking assembly **300** and the central tube **170** as the central tube **170** is moved in a distal or proximal direction. The central tube **170** can be secured to the anchoring surface **308** such as by thermal bonding, adhesive bonding, crimping, or any of a variety of other securing techniques known in the art.

Additionally or alternatively, the locking assembly **300** can include a second lumen **312.** The second lumen **312** is a passage between the distal face **302** and the proximal face **304** of the housing **301.** However, in other configurations, the second lumen **312** may communicate only with the distal face **302,** and not with the proximal face **304,** of the housing **301.** The second lumen **312** can be configured to retain a second elongate member (e.g., the contralateral guidewire 194) to the locking assembly 300. The contralateral guidewire 194 can be released from the second lumen **312** upon activation of the locking assembly **300** by a user.

The locking assembly **300** can include a first recess **314** that extends from a lateral wall **316** of the housing **301** toward the longitudinal axis **306** of the locking assembly **300.** The second lumen **312** can communicate with the first recess **314.** Additionally or alternatively, the first lumen **310** can communicate with the first recess **314.** The locking assembly **300** can include a second recess **320** that extends from the lateral wall **316** of the housing **301** toward the longitudinal axis **306** of the locking assembly **300.** The locking assembly **300** can include a divider **322** that is interposed between the first recess **314** and the second recess **320.** The locking assembly **300** can include a through-hole **324** that extends through the divider **322** and communicates between the first and second recesses **314, 320** to form a recessed portion.

A proximal portion **326** of the locking assembly **300** can be tapered. The proximal portion **326** can be tapered so that the transverse cross-sectional area of the proximal portion **326** decreases along the proximal direction. The proximal portion **326** can be tapered to allow the proximal portion **326** to be withdrawn out of the graft **50** and back into the outer sheath **128** without having the locking assembly **300** getting caught on the graft **50** or the outer sheath **128** or any other intervening structure. The locking assembly **300** can have a taper angle defined as the angle between the longitudinal axis **306** of the locking assembly **300** and the lateral wall of the proximal portion of the locking assembly **300.** The taper angle can be between about 15 and 60 degrees, between about 20 and 45 degrees, and between about 25 and 35 degrees. The taper angle can be 30 degrees with a tolerance of 1 degree.

A distal portion **330** of the locking assembly **300** can include a protrusion **332,** extending radially outward from housing **301.** The protrusion **332** may circumferentially surround the entire distal portion **330** of the locking assembly **300,** or may surround only a portion of the distal portion **330** of the locking assembly **300.** The protrusion **332** may extend from only a portion of the distal portion **330.** The second lumen **312** can be interposed between the protrusion **332** and the first lumen **310.** The protrusion **332** can be configured to provide strain relief to an elongate member that extends distally from the second lumen **312** and then bends back in the proximal direction. The protrusion **332** can have a radius of curvature between about 0.005 and 0.1 inches, between about 0.01 and 0.05 inches, and between about 0.015 and 0.025 inches. The protrusion **332** can have a radius of curvature of 0.02 inches with a tolerance of 0.01 inches, wherein 1 inch corresponds to 25.4 millimeters.

The locking assembly **300** can have a length dimension that defines the distance between the proximal and distal faces **304, 302.** The length dimension can be between about 0.1 and 1.0 inches, between about 0.2 and 0.5 inches, and between about 0.3 and 0.4 inches. The length dimension can be 0.375 inches with a tolerance of 0.010 inches. The locking assembly **300** can have a width dimension perpendicular to the length dimension. The width dimension can be between about 0.05 and 0.5 inches, between about 0.1 and 0.3 inches, and between about 0.15 and 0.2 inches. The width dimension can be 0.187 inches with a tolerance of 0.002 inches. The locking assembly **300** can have a first aspect ratio defined as the length dimension divided by the width dimension. The first aspect ratio can be between about 0.5 and 5, between about 1and 3, and between about 1.75 and 2.25. The first aspect ratio can be 2.0.

The first lumen 310 of the locking assembly 300 can have a diameter of between about 0.01 and 0.2 inches, between about 0.02 and 0.1 inches, and between about 0.04 and 0.06 inches. The first lumen 310 can have a diameter of 0.055 inches with a tolerance of 0.002 inches. The second lumen **312** of the locking assembly **300** can have a diameter of between about 0.01 and 0.1 inches, between about 0.02 and 0.05 inches, and between about 0.03 and 0.04 inches. The second lumen **312** can have a diameter of 0.033 inches with a tolerance of 0.002 inches. The locking assembly **300 can** have a second aspect ratio defined as the diameter of the first lumen **310** divided by the diameter of the second lumen **312.** The second aspect ratio of the locking assembly **300** can be between about 1 and 3, between about 1.5 and 2. The second aspect ratio of the locking assembly **300** can be 1.667. The center points of the first and second lumens can be separated from one another by a spacing dimension. The spacing dimension can be between about 0.04 and 0.07 inches, and between 0.05 and 0.06 inches. The spacing dimension can be 0.053 inches with a tolerance of 0.002 inches.

The locking assembly **300** can include an elastomeric member **334.** The elastomeric member **334** may occupy at least a portion of the first recess **314.** The elastomeric member **334** can have an outer surface **336** that is flush with at least a portion of the lateral wall of the locking assembly **300.** The first recess **314** can be configured to retain the elastomeric member **334** within the locking assembly **300.** Additionally or alternatively, the elastomeric member **334** may occupy at least a portion of the second recess **320.** The second recess **320** can be configured to retain the elastomeric member **334** within the locking assembly **300.** The elastomeric member **334** can be configured to span the divider **322.** The elastomeric member **334** can have a first portion that resides in the first recess **314** while a second portion of the elastomeric member **334** resides in the second recess **320,** the first and second portions of the elastomeric member **334** being connected by a segment of the elastomeric member **334** that extends through the through-hole **324.**

The elastomeric member **334** can be configured to enhance the ability of the second lumen **312** to retain the contralateral guidewire **194** of the delivery catheter **120.** For example, the elastomeric member **334** can be configured to intrude into at least a portion of the second lumen **312.** The locking assembly **300** can be configured so that at least a portion of the elastomeric member **334** can interface with an elongate member inserted into the second lumen **312.** The elastomeric member **334** may form a friction fit with an elongate member inserted into the second lumen **312,** helping to retain the elongate member in the second lumen **312.** Different non-limiting exemplary embodiments of the elastomeric member 334 are discussed below.

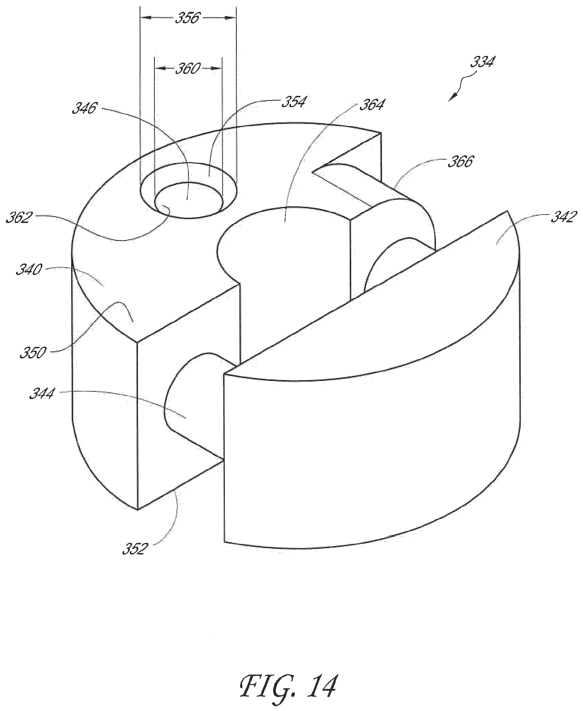

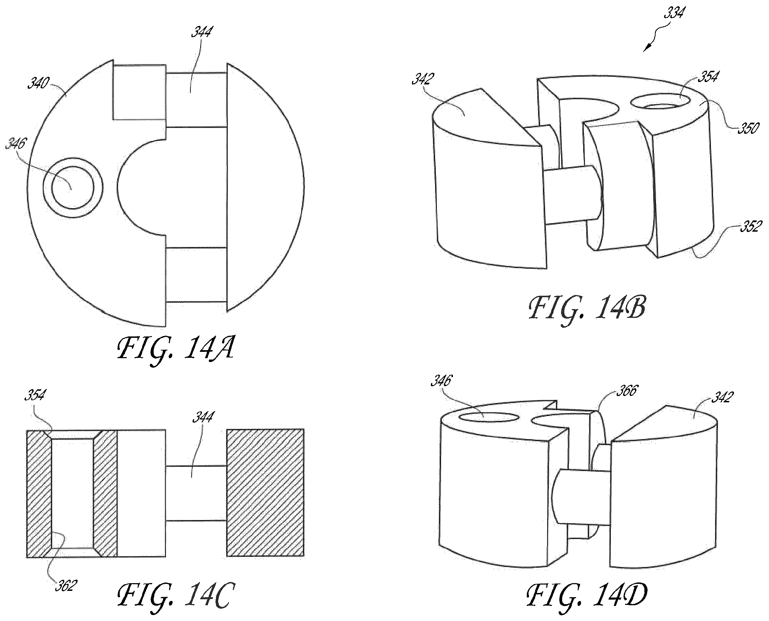

FIG. 14 shows an isometric view of one exemplary embodiment of the elastomeric member **334.** The elastomeric member **334** can include a first portion **340** that is configured to be retained within the first recess **314** of the locking assembly **300.** Additionally or alternatively, the elastomeric member **334** may include a second portion **342** that is configured to be retained within the second recess **320** of the locking assembly **300.** The first portion **340** can be connected to the second portion **342** by at least one segment **344.** In the non-limiting exemplary example depicted in FIG. 14 the elastomeric member **334** can include a first portion **340** joined to a second portion **342** by two segments **344,** the segments **344** being cylindrical in shape. The elastomeric member **344** can include a segment **344** having a shape other than cylindrical. The second portion **342** and the segment **344** can be configured to enhance retention of the elastomeric member **334** within the housing **301** of the locking assembly **300.** The second portion **342** can provide a mechanical lock between the elastomeric member **334** and the housing **301** of the locking assembly **300,** thereby increasing the strength of the attachment between the elastomeric member **334** and the housing **301.**

The elastomeric member **334** can include a retention portion **346** configured to retain a second elongate member **305** that is inserted into the second lumen **312** of the locking assembly **300.** The retention portion **346** can define an opening **348** (e.g., through-hole, lumen, or otherwise) that extends at least partially through the elastomeric member **334,** e.g., between a distal face **350** and a proximal face **352** of the elastomeric member **334** or only in communication with the distal face **350** of the elastomeric member **334.** The opening **348** can be concentric with the second lumen **312** of the locking assembly **300** when the elastomeric member **334** is seated within the first recess **314** of the locking assembly **300.**

An end portion of the opening **348** has a diameter **356** that can be larger than an intermediate diameter **360** of an intermediate portion **362** of the retention portion **346.** The end portion **354** of the retention portion **346** can be configured to guide an elongate member into the intermediate portion **356** of the retention portion **346.** The end portion **354** of the retention portion **346** can include a canted wall that funnels an inserted elongate member into the intermediate portion **356** of the retention portion **346.**

The elastomeric member **334** can include a passageway defined by a curved surface **364** that aligns with the first lumen **310** of the locking assembly **300.** The curved surface **364** of the elastomeric member **334** can interface with (e.g., by bonding) the housing **301** of the locking assembly **300.** The housing **301** of the locking assembly can interface with (e.g., by welding) to the elongate member that passes through the first lumen of the locking assembly **300.**

The elastomeric member **334** can include a curved portion **366.** The curved portion **366** can be configured to enhance the bonding between the elastomeric member **334** and the housing **301** of the locking assembly **300.** FIG. 14A is a top view of the elastomeric member **334** depicted in FIG. 14. FIG. 14B is an offset rear view of the elastomeric member **334** depicted in FIG.14, showing the curved portion **366** in more detail. FIG. 14C is a front cross-sectional view of the elastomeric member **334** depicted in FIG. 14. FIG. 14D is an offset front view of the elastomeric member **334** depicted in FIG. 14.

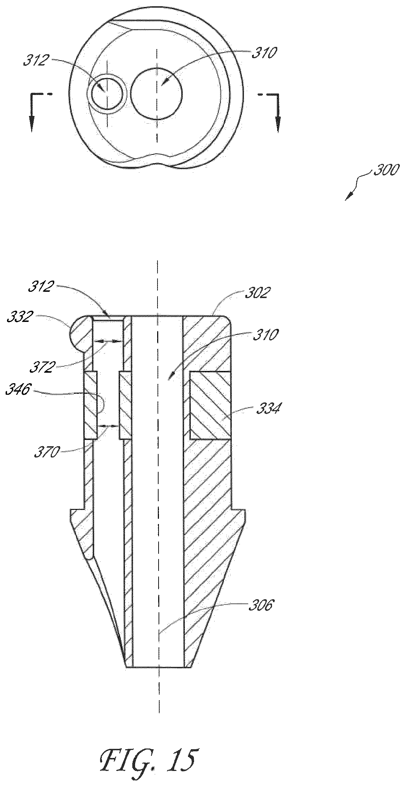

FIG. 15 depicts a cross-sectional view of an exemplary embodiment of the locking assembly **300.** The elastomeric element **334** can include a retention portion **346** that at least partially aligns with the second lumen **312.** The retention portion **346** can include an opening **348** having a width **370.** The width **370** of the passageway of the retention portion **346** can be smaller than the width **372** of the second lumen **312,** thereby causing at least a portion of the retention portion **346** to intrude upon the second lumen **312.** The retention portion **346** can define an opening **348** that partially aligns with the second lumen **312.** For example, the retention portion **346** may intrude upon the second lumen **312** from only one side. In some configurations, the retention portion **346** can be cup-shaped, with the mouth of the cup-shaped retention portion 346 facing the distal surface **302** of the locking assembly **300.**

As discussed, in some embodiments, the retention portion **346** can include an opening **348** formed within the elastomeric member **334.** The elastomeric member **334** can be made of an elastic material such as silicone. The opening **348** of the retention portion **346** can stretch and/or compress to accommodate an elongate member inserted into the retention portion **346.** The retention portion **346** can be configured to form a friction fit with an elongate member inserted into the opening **348** of the retention portion **346.** The friction fit between the retention portion **346** and the elongate member can resist distal movement of the elongate member relative to the retention portion **346** until the elongate member is subjected to sufficient tension in the distal direction.

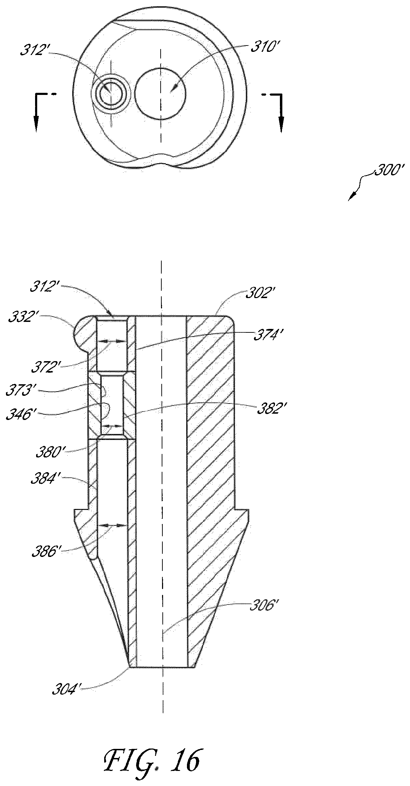

FIG. 16 depicts a cross-sectional view of a non-limiting alternative embodiment of the locking assembly **300'** that includes the housing **301'** but not include an elastomeric member **334'.** The housing **301'** can be substantially similar to that described above except without a recessed portion configured to receive the elastomeric member. In this embodiment, the locking assembly **300'** can include a retention portion **346'** which can include the second lumen **312'.** The second lumen **312'** can have a uniform cross-sectional area or a non-uniform cross-sectional area (e.g., a constriction of the second lumen **312').** A distal portion **374'** of the second lumen **312'** can have a width **376'** that is greater than a width **380'** of an intermediate portion **382'** of the second lumen **312'.** The second lumen **312'** may include a proximal portion **384'** that is proximal of the intermediate portion **382'** of the second lumen **312'.** The proximal portion **384'** can have a width **386'** that is greater than the width **380'** of the intermediate portion **382'.** The operation of the retention portion **346'** is discussed below.

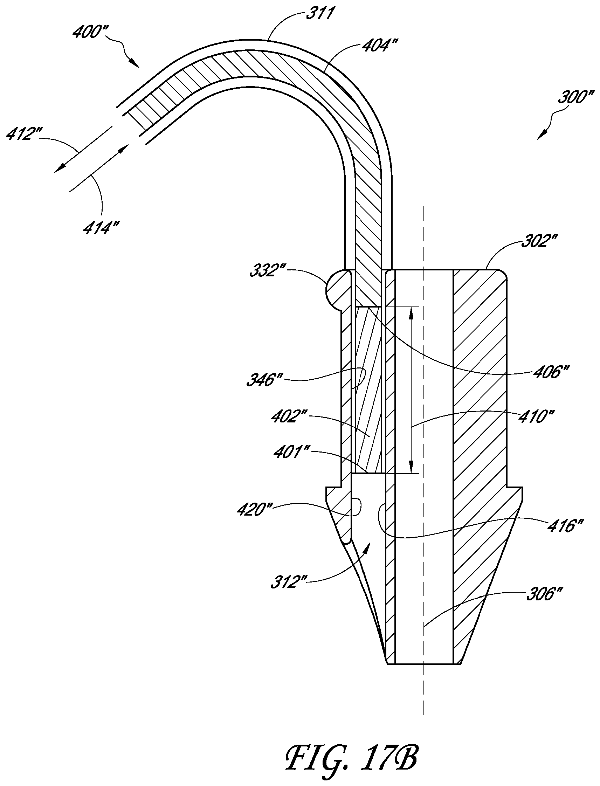

Figures 17A and 17B schematically illustrate a method of releasing the guidewire from the locking assembly 300 that can be used in connection with both the locking assemblies with and without the elastomeric member (shown in FIGS. 15 and 16). Although these figures illustrate the second lumen **312"** having a generally uniform diameter, the diameter may vary as shown in FIG. 16. FIG. 17A depicts a non-limiting exemplary embodiment of the locking assembly **300"** with a distal portion **194b** (also called the locking portion) of the contralateral guidewire **194** inserted into the second lumen **312"** of the locking assembly **300".** When the contralateral guidewire **194** is inserted into the locking assembly **300",** general advancement and retraction of the contralateral guidewire **194** without the appropriate vertical force and/or actuation will not release the contralateral guidewire **194** from the locking assembly **300".**

The contralateral guidewire **194** can include multiple regions, with each region having a different stiffness. For example, the stiffness of the locking portion **194b** of the contralateral guidewire **194** can be selected to be higher than the stiffness of a proximal portion **194a** (also called the floppy region) of the contralateral guidewire **194. 194194194194**By designing the floppy region **194a** to have a low stiffness, the a proximal portion **194a** of the contralateral guidewire **194** will be sufficiently flexible to avoid causing damage to surrounding tissue. The tensile strength of the floppy region **194a** can be greater than about 1 lbf, greater than about 2 lbf, greater than about 6 lbf, and greater than about 8 lbf.

The locking portion **194b** of the contralateral guidewire **194** can extend from the floppy region **194a** of the contralateral guidewire **194** at an interface **406"**. The length **410"** of the locking portion **194b** can be selected from different lengths. In some embodiments, the length of the locking portion **194b"** can be between about 0.3 and 0.8 cm. Additionally or alternatively, the insertion depth of the locking portion **194b** into the second lumen **312"** can be adjusted. The contralateral guidewire **194** and the locking assembly **300"** may be tailored so that the interface **406"** can be located distal, proximal, or co-planar to the distal face **302"** of the locking assembly **300".**

During use, a user may pull on the contralateral guidewire **194,** creating a tension **412"** in the contralateral guidewire **194,** thereby causing the contralateral guidewire **194** to bend in the proximal direction, as illustrated in FIG. 17A. Additionally or alternatively, a user may push on the contralateral guidewire **194,** causing a compressive force **414"** that buckles the contralateral guidewire **194** in the distal direction, thereby pushing the locking portion **194b** into the retention portion **346".** The locking assembly **300"** can include a protrusion **332"** that provides strain relief to the contralateral guidewire **194** when the contralateral wire **194** bends back in the proximal direction. The position of the interface **406"** relative to the distal face **302"** of the locking assembly **300"** can be selected so that as the tension **412"** pulls on the interface **406",** the locking portion **194b** of the contralateral guidewire **194** is retained against a lateral surface **416"** of the retention portion **346",** thereby preventing the contralateral guidewire **194** from decoupling from the locking assembly **300".** The clearance between the locking portion **194b** and the lateral surface **416"** can also be selected to further define the tension **412"** required to decouple the contralateral guidewire **194** from the locking assembly **300".** The locking assembly **300"** can be configured to release the locking portion **194b** of the contralateral guidewire **194** when a force is applied to an intermediate portion of the contralateral guidewire **194** at an angle of less than or equal to about 60 degrees from the locking portion **194b** of the contralateral guidewire **194** and/or at least about 45 degrees from the locking portion **194b"** of the contralateral guidewire **194".**

FIG. 17B shows an illustrative embodiment of a locking assembly **300"** that is configured to release the contralateral guidewire **194** upon a release member **311** (e.g., pigtail catheter) contacting with the locking assembly **300"** and applying a vertical force to draw the locking portion **194b** of the contralateral guidewire **194** out of the retention portion **346".** In the non-limiting exemplary locking assembly **300"** depicted in FIG. 17B, the release member **311** can be a release sheath that is configured to be disposed over the contralateral guidewire **194.** The release member **311** may completely circumferentially surround the contralateral guidewire **194,** or the release member **311** may only partially circumferentially surround the contralateral guidewire **194.** The release member **311** may be composed of a plurality of segments. The segments may be identical to one another or different from one another. Some, all, or no segments may completely circumferentially surround the contralateral guidewire **194** while some, all, or no other segments only partially surround the contralateral guidewire **194.** The segments may be separated from one another by one or more hinge points. The hinge points may be configured to allow the segments to bend, flex, or pivot relative to one another.

The release member **311** can be introduced at the contralateral access site by passing the distal end of the release member **311** over the proximal end of the contralateral guidewire **194.** The release sheath **420"** can be advanced over the contralateral guidewire **194** until the distal face of the release member **311** engages the distal face **302"** of the locking assembly **300".** The outer diameter of the distal face of the release member **311** can be selected so that the distal face abuts against the distal face **302"** of the locking assembly. As a user applies a compressive force **414"** to the release member **311** and contralateral guidewire **194,** the release member **311** buckles in the distal direction. As the release member **311** buckles (loops, forms a U-shape, or otherwise bends) in the distal direction, the portion of the contralateral guidewire **194** that distally extends from the retention member **346"** of the locking assembly **300"** is aligned to be substantially perpendicular to the distal face **302"** of the locking assembly **300".** A user may now apply tension **412"** to the contralateral guidewire **194.** Once a distally extending portion of the contralateral guidewire **194** is longitudinally aligned with the retention member **346",** the **194**the contralateral guidewire **194** is released from the retention member **346".** The locking assembly **300"** can be configured to retain the contralateral guidewire **194** until a vertical force of at least 0.1 lbf (or at least about 0.5 lbf, at least about 1.0 lbf, or otherwise) is applied to the contralateral guidewire **194.**

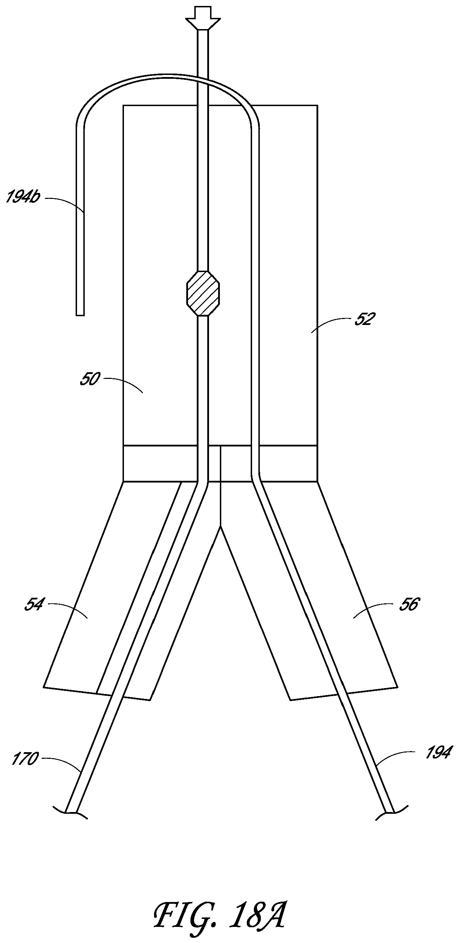

The reversible coupling of the contralateral guidewire **194** to the locking assembly 300 can be accomplished by alternative embodiments that are within the scope of the present disclosure. For example, the locking assembly **300"** depicted in FIGS. 17A-B can include an elastomeric member **334,** as discussed above. Additionally or alternatively, the locking assembly **300** can include any of the features depicted in the alternative embodiments depicted in FIGS. 18A-R.

FIG. 18A depicts an embodiment where the locking portion **194b** of the contralateral guidewire **194** is retained by wrapping the locking portion **194b** of the contralateral guidewire **194** over the main branch portion **52** of the graft **50.** The locking portion **194b** can be secured to the outer surface of the main branch portion **52** by a sheath (not shown) that is deployed by a suture (not shown) as described above for the deployment of the main branch portion **52** of the graft **50.** The locking portion **194b** may be larger in diameter, longer, and/or more rigid than remaining portions of the contralateral guidewire **194.** The sheath that secures the locking portion **194b** can be the main branch sheath **186** or a sheath different from the main branch sheath **186.**

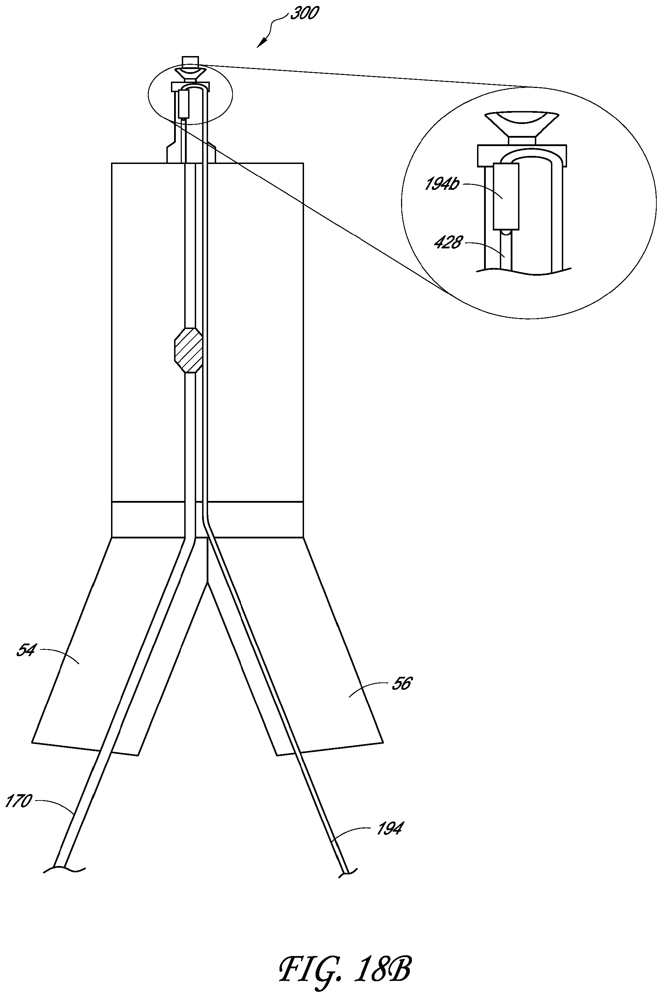

FIG. 18B depicts an embodiment of the locking assembly **300** that can have a track **428** configured to retain the locking portion **194b** of the contralateral guidewire **194.** The locking portion **194b** may be larger and/or more rigid than a remaining portion of the contralateral guidewire **194** to help retain the locking portion **194b** within the track **428.** The track **428** may be an open faced channel having a variable width so that when tension is applied to the contralateral guidewire **194** the locking portion **194b** is drawn up into a necked region of the track **428,** thereby constraining the locking portion **194b** from escaping the track **428.** Additionally or alternatively, a region of the track **428** may be an open faced channel having an enlarged region where the width of the track **428** is larger than the width of the locking portion **194b.** A user may free the contralateral guidewire **194** from the locking assembly **300** by advancing the locking portion **194b** of the contralateral guidewire **194** until the locking portion **194b** is aligned with the enlarged region of the track **428,** thereby allowing the locking portion **194b** to escape the track **428.**

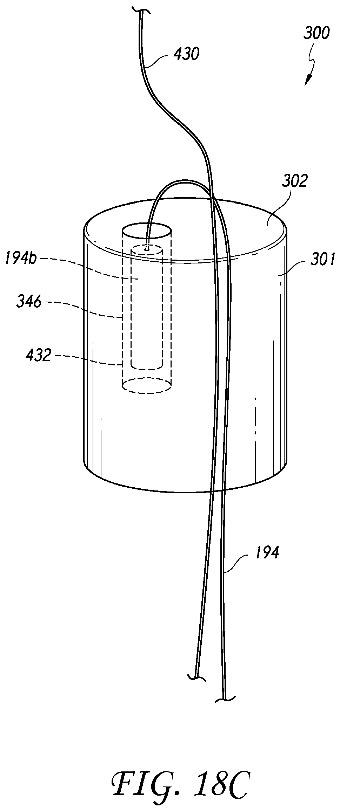

FIG. 18C depicts an embodiment of the locking assembly **300** that has a retaining wire **430.** The retaining wire **430** can be configured to hold the contralateral guidewire **194** against the distal face **302** of the locking assembly **300,** thereby keeping the locking portion **194b** within the retention portion **346** of the locking assembly **300.** The retention portion **346** can be a groove on the side of the housing **301** of the locking assembly **300.** The retention portion **346** may be a pocket **432** that is surrounded by the housing **301.** The width of the groove or pocket **432** may vary as described above. The retaining wire **430** can be configured to be withdrawn from the locking assembly **300,** thereby allowing the locking portion **194b** to potentially release from the pocket **432.**

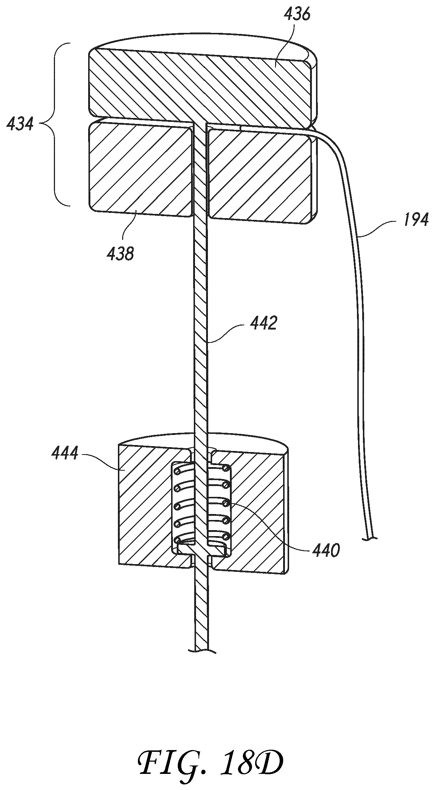

FIG. 18D depicts an embodiment of the locking assembly **300** that has a clamp **434.** The top portion **436** of the clamp **434** may be pulled against the bottom portion **438** of the clamp **434** by a spring **440** that is connected to the top portion **436** by a tension element **442.** The contralateral guidewire **194** may be coupled to the locking assembly **300** by virtue of being compressed between the top and bottom portions **436, 438** of the clamp **434.** A user may activate a trigger **444** to compress the spring **440** reducing the tension element, thereby reducing the compressive force between the top and bottom portions **436, 438** of the clamp **434** and allowing the contralateral guidewire **194** to decouple from the locking assembly **300.** In other embodiments, the tension in the spring 440 may be released by moving the trigger 444 or by releasing the tension on the spring 440.

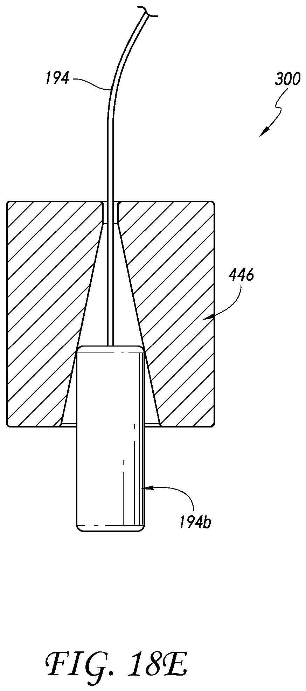

FIG. 18E depicts an embodiment of the locking assembly **300** having a duckbill valve **446.** The duckbill valve **446** can be configured to retain a locking portion **194b** at the distal end of the contralateral guidewire **194.** The locking portion **194b** may be larger and/or more rigid than a remaining portion of the contralateral guidewire **194.** A user may decouple the contralateral guidewire **194** from the locking assembly **300** by advancing the contralateral guidewire **194** to allow the locking portion **194b** to escape the duckbill valve **446.**

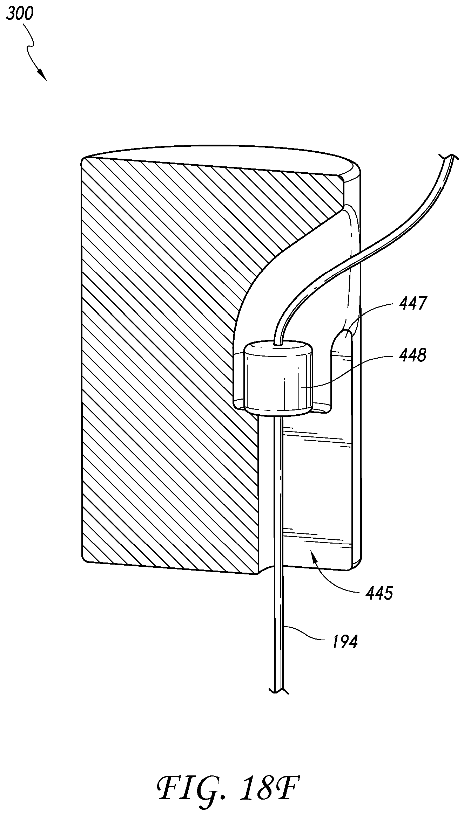

FIG. 18F depicts an embodiment of the locking assembly **300** configured to retain a bead **448** attached to the contralateral guidewire **194.** The bead **448** can interface with (e.g., by bonding) the contralateral guidewire **194.** As shown in FIG. 18F, the bead **448** can sit within a channel **445** that has a narrow proximal portion and a wider distal portion. The narrow proximal portion of the channel **445** can be configured to prevent the bead **448** from being pulled proximally through the channel. The wider portion of the channel can have an overhang **447** that can be configured to prevent the bead **448** from leaving the channel **445** unless the bead **448** is advance sufficiently far distally to clear the overhang **447.**

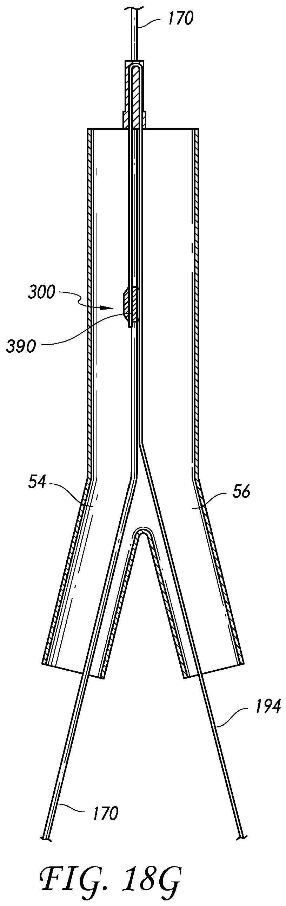

FIG. 18G depicts an embodiment of the locking assembly **300** having a plastic retention member **390** (e.g., soft wing) that can be located within the main branch portion **52** of the graft **50.** The plastic retention member **390** can interface (e.g., by friction, press fit, molded, adhered, thermally bonded, mechanically locked, otherwise secured) with the central tube **170.** The contralateral guidewire **194** can extend distally through the graft **50,** pass over or through a housing member and bend proximally back into the graft **50** to terminate at the plastic retention member **390.** The locking portion **194b** of the contralateral guidewire **194** can be configured to be retained by the plastic retention member **390** as described above in FIG. 18B.

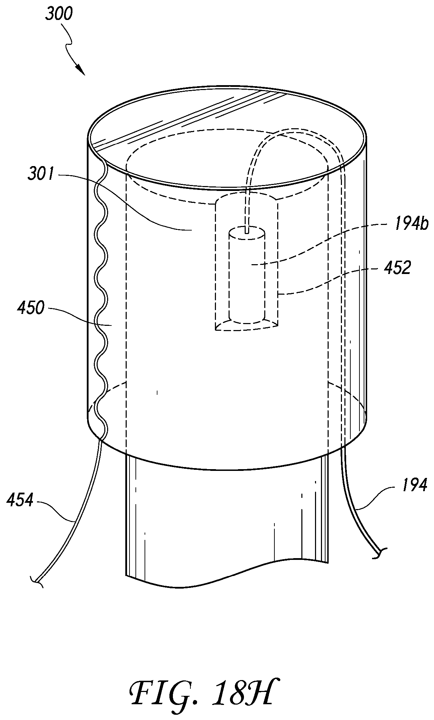

FIG. 18H depicts an embodiment of the locking assembly **300** having a wrapper **450.** The wrapper **450** can enclose at least a portion of the housing **301** of the locking assembly **300.** The wrapper **450** can be configured to restrain the locking portion **194b** of the contralateral guidewire **194** within a channel **452** formed in the side of the housing **301.** The wrapper **450** can be removed by a control suture **454,** thereby allowing the locking portion **194b** to escape the channel **452.**

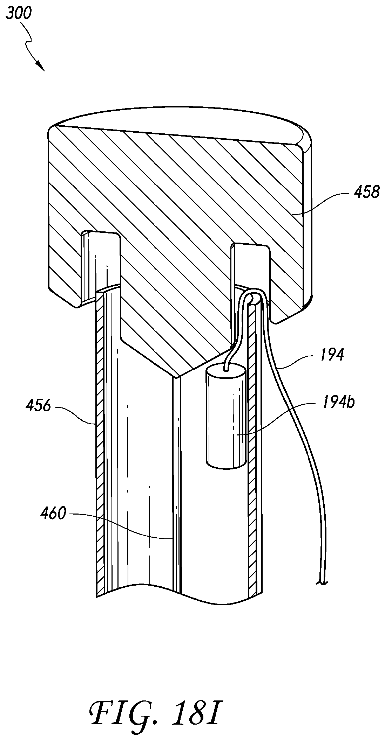

FIG. 18I depicts an embodiment of the locking assembly **300** having a rigid sheath **456.** The rigid sheath **456** can be configured to press the contralateral guidewire **194** against an abutment **458,** thereby locking the contralateral guidewire **194** to the locking assembly **300.** The contralateral guidewire **194** may be decoupled from the locking assembly **300** by proximally withdrawing the rigid sheath **456** or distally advancing the abutment **458.** A control rod **460** attached to the abutment **458** can allow a user to distally advance the abutment **458.** The locking portion **194b** may be larger and/or more rigid than a remaining portion of the contralateral guidewire **194** locking portion

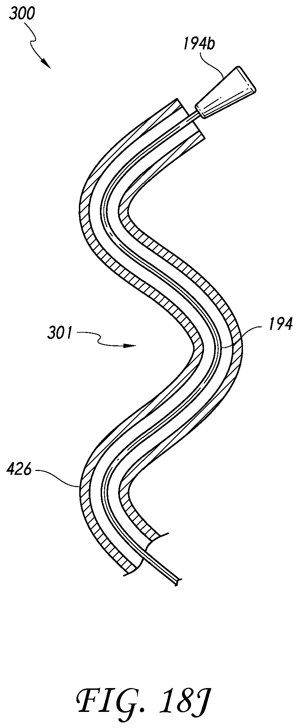

FIG. 18J depicts an embodiment of the locking assembly **300** having a curved groove **462.** The curved groove **462** may be S-shaped, U-shaped, sinusoidal, zig-zag-shaped or combinations thereof. The curved groove **462** may be formed in the outer surface of the housing **301** of the locking assembly **300** described above. The contralateral guidewire **194** may include a locking portion **194b** that has a width or length that prevents the locking portion **194b** from being pulled through the curved groove **462.** The locking assembly **300** can be configured so that when the contralateral guidewire **194** is advanced distally the locking portion **194b** of the contralateral guidewire **194** may peel out of the housing **301,** thereby freeing the contralateral guidewire **194** from the locking assembly. Additionally or alternatively, the locking assembly **300** may be configured so that a release member **311** is advanced over the contralateral guidewire **194** to assist in freeing the contralateral guidewire **194** from the locking assembly **300.**

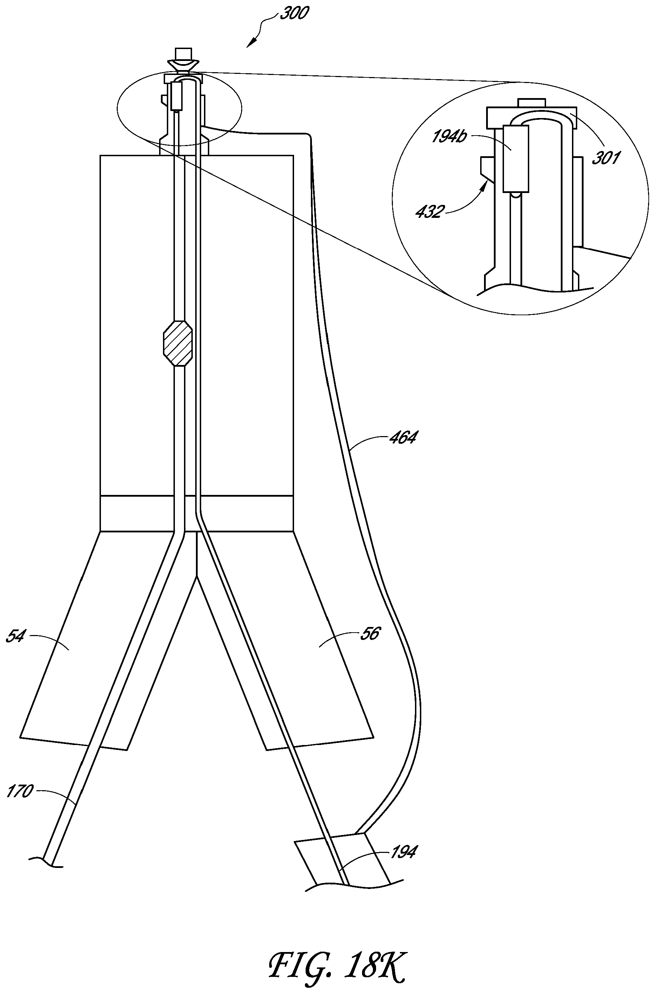

FIG. 18K depicts an embodiment of the locking assembly **300** having an extended portion **464** of the contralateral branch sheath **190.** The extended portion **464** may wrap around a portion of the locking assembly **300,** thereby retaining the locking portion **194b** of the contralateral guidewire **194** within a pocket **432** of the locking assembly **300.** The extended portion **464** may be held closed by a suture. The suture can be coupled to the contralateral branch sheath **190.** The suture can be configured to separate the extended portion **464** from the housing **301** as the contralateral branch sheath **190** is proximally withdrawn, thereby freeing the locking portion **194b** of the contralateral guidewire 194 from the locking assembly **300.**

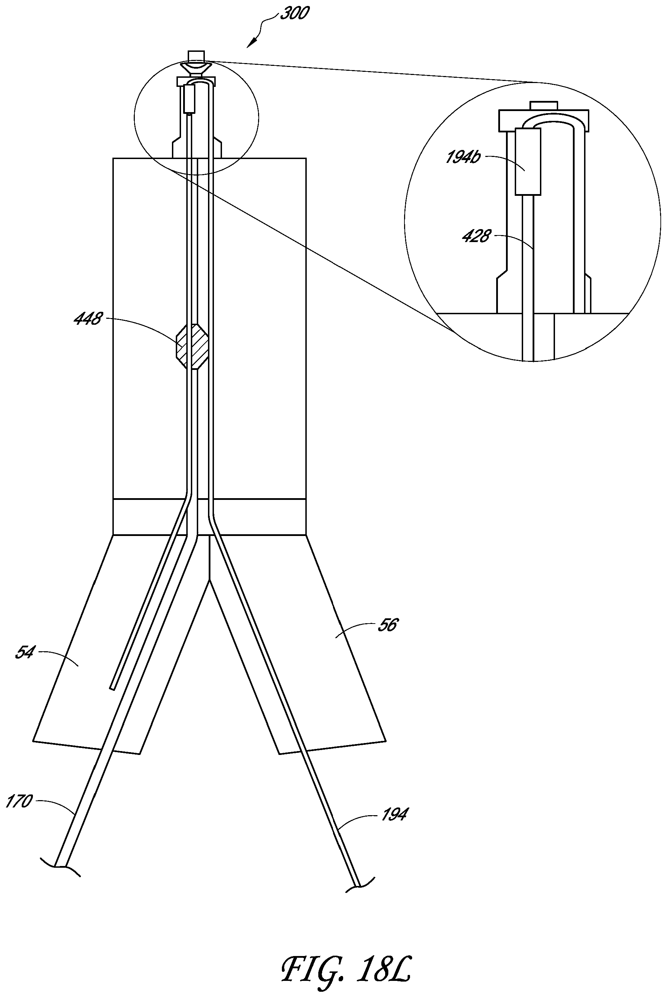

FIG. 18L depicts an embodiment of the locking assembly **300** having a contralateral guidewire **194** that extends into the ipsilateral branch portion **54** of graft **50.** The ipsilateral limb pinches, compresses, or otherwise provides friction such that the guidewire when inadvertently advanced does not disconnect from the catheter until the ipsilateral branch portion is deployed. The contralateral guidewire **194** may extend through a bead **448.** The bead **448** may be configured to be retained within a channel 452 formed in the side of or through the housing **301** of the locking assembly **300** as described above.

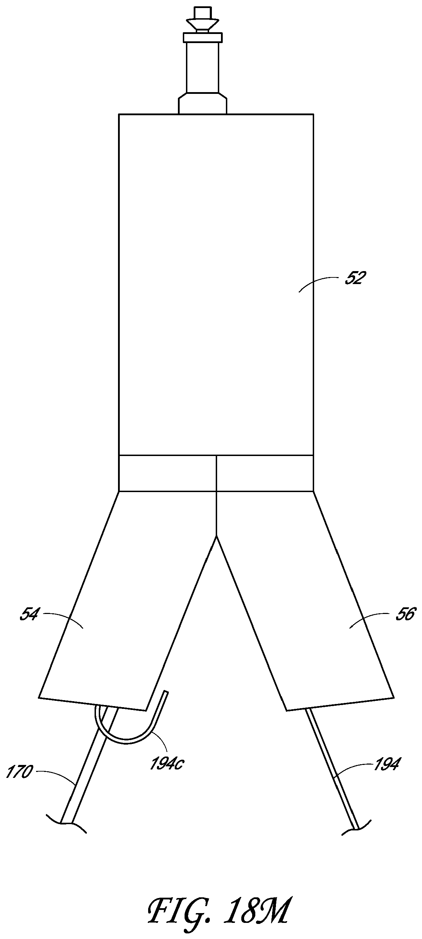

FIG. 18M depicts an embodiment of the locking assembly **300** having a folded portion **194c** of the contralateral guidewire **194** that is folded over the ipsilateral branch portion **54** of the graft **50.** The folded portion **194c** may be held against an outer surface of the ipsilateral branch portion **54** by the ipsilateral branch sheath **188.** Retraction of the ipsilateral branch sheath **188** may then free the folded portion **194c,** thereby allowing the contralateral guidewire **194** to be removed from the locking assembly **300.**

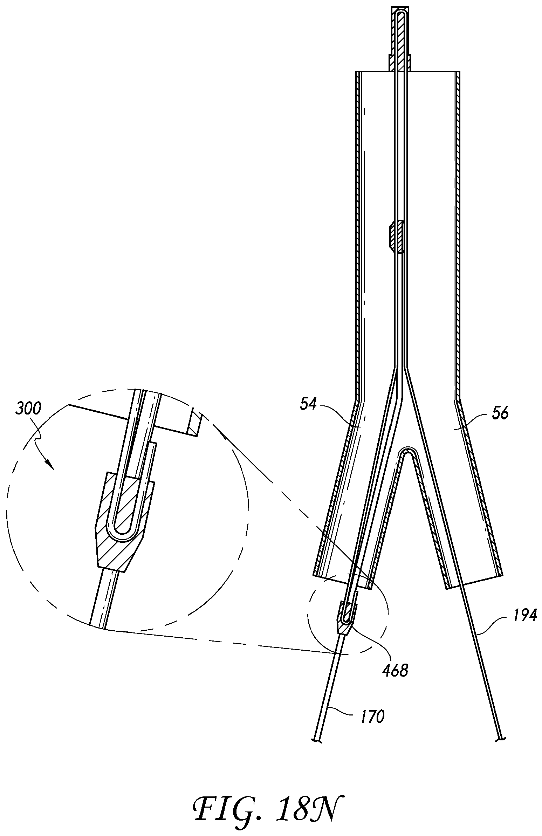

FIG. 18N depicts an embodiment of the locking assembly **300** having ipsilateral locking member **468.** The ipsilateral locking member **468** can include a channel **452** configured to retain a locking portion **194b** or bead **448** that is coupled to the contralateral guidewire **194,** as discussed above.

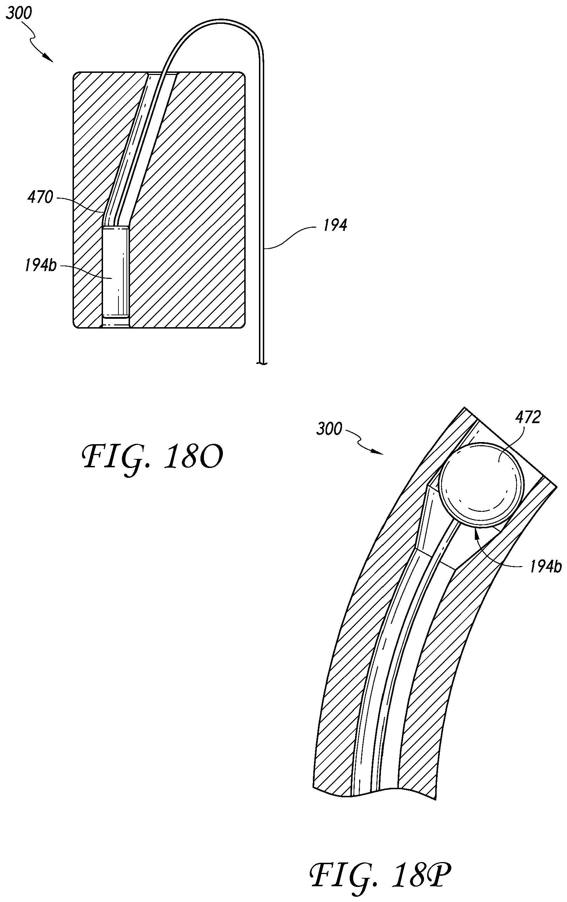

FIG. 18O depicts an embodiment of the locking assembly **300** having a bent channel **470.** The bent channel **470** can be configured so that the locking portion **194b** of the contralateral guidewire **194** is unable to navigate through the bent channel **470.** The locking portion **194b** of the contralateral guidewire **194** can be decoupled for the locking assembly **300** by advancing the locking portion **194b** proximally through the bent channel **470.**

FIG. 18P depicts an embodiment of the locking assembly **300** having a ball feature **472** at the locking portion **194b** of the contralateral guidewire **194.** The ball feature **472** can be configured to function similar to the bead **448** described above for FIG. 18F. The ball feature **472** may be configured to be too large to pass proximally through the channel of the locking assembly **300.** The ball feature can be configured so that the locking portion **194b** is freed from the locking assembly when the ball feature is advanced distally, thereby allowing the contralateral guidewire **194** to peel out of the channel in the locking assembly **300.**

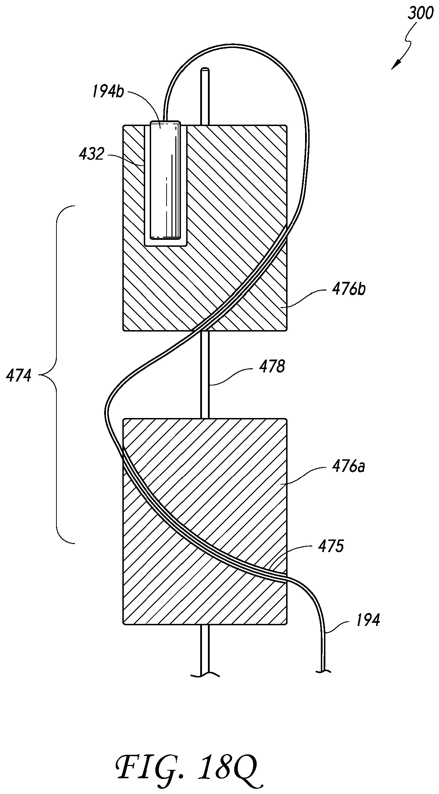

FIG. 18Q depicts an embodiment of the locking assembly **300** having a segmented locking channel **474.** The segmented locking channel **474** may include a plurality of segments **476 a, b,** each having a groove **475** formed into the surface of the segment **476 a, b.** The segments **476 a,b** may be joined together by a coupling member **478**. The contralateral guidewire **194** may navigate through the segmented locking channel **474.** The locking portion **194b** of the contralateral guidewire **194** may be retained within a pocket **432** that is formed in one of the segments **476b** of the plurality of segments **476 a,b.**

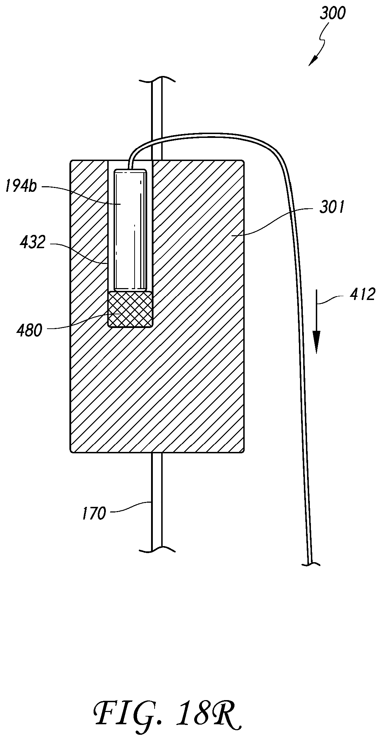

FIG. 18R depicts an embodiment of the locking assembly **300** having a magnetic component **480.** The magnetic component **480** can be disposed within the pocket **432** formed into the housing **301** of the locking assembly **300.** The locking portion **194b** of the contralateral guidewire **194** may include magnetic material that causes the locking portion **194b** to be magnetically attracted to the magnetic member **180,** thereby resisting upward forces **412** on the contralateral guidewire **194** from decoupling the locking portion **194b** of the contralateral guidewire **194** from the locking assembly **300.**

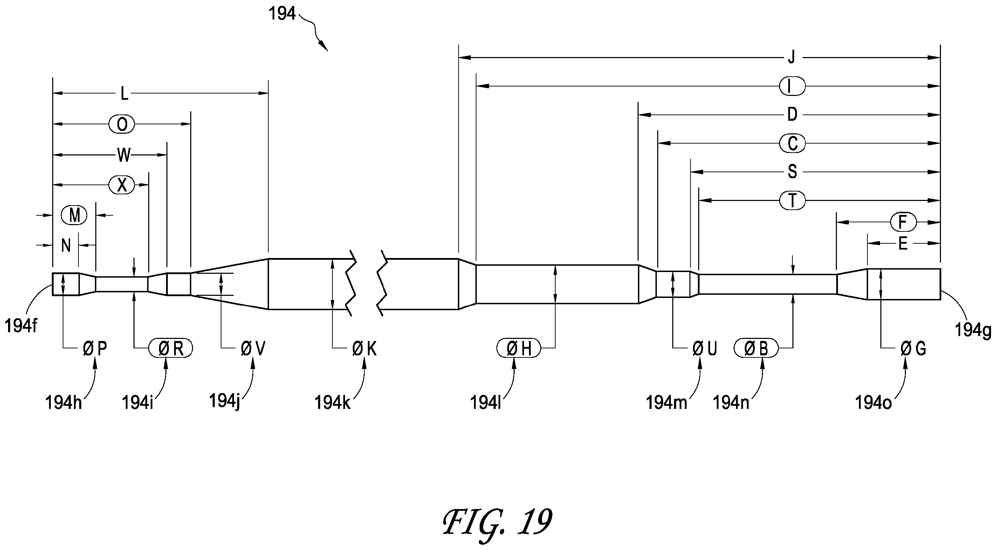

FIG. 19 depicts a non-limiting exemplary embodiment of the contralateral guidewire **194.** The contralateral guidewire **194** can be configured to include multiple regions that are joined together. The contralateral guidewire **194** can have a first end **194f** and a second end **194g**. The serial arrangement of the multiple regions of the contralateral guidewire can be varied according to the needs of the user. The serial arrangement of the regions of the contralateral guidewire **194** shown in FIG. 19 is illustrative only and not to be taken as limiting. The contralateral guidewire **194** can include a wire core, shrink tube made of suitable material (e.g., PTFE), adhesive, alloys containing platinum, and combinations thereof. The platinum alloys can contain 90% platinum and 10% irradium, or 92% platinum and 8% tungsten.

The contralateral guidewire **194** may include a distal coil made of wire containing platinum, e.g., at least about 90% platinum, at least about 92% platinum, at least about 95% platinum, or at least about 99% platinum. In some embodiments, the contralateral guidewire **194** can be constructed from about 92% platinum and 8% tungsten. The distal coil may be made of wire having an outer diameter of 0.003 inches. The distal coil may have a pitch of 0.003 inches. The distal coil may have a coil outer diameter of 0.025 inches. The distal coil may have a coil length of 8.0 cm with a tolerance of 0.3 cm.

The contralateral guidewire **194** may include a proximal coil made of wire containing 92% platinum and 8% tungsten. The proximal coil may be made of wire having an outer diameter of 0.003 inches. The proximal coil may have a pitch of 0.003 inches. The proximal coil may have a coil outer diameter of 0.025 inches. The proximal coil may have a coil length of 15.0 cm with a tolerance of 0.3 cm.

A first region **194h** (denoted as having a diameter of P in FIG. 19) of the contralateral guidewire **194** can have a diameter of 0.0160 inches with a tolerance 0.0002 inches. The first region **194h** can have a length of 0.3 cm with a tolerance of 0.1 cm. A second region **194i** (denoted as having a diameter of R in FIG. 19) of the contralateral guidewire **194** can have a smaller diameter than the first region **194h,** e.g., less than or equal to one-half the diameter of the first region **194h.** For example, the diameter of the second region **194i** can be about 0.0080 inches with a tolerance 0.0002 inches. The second region **194i** can be longer than the first region **194h.** For example, the second region **194i** can have a length of 10.4 cm with a tolerance of 0.1 cm. A third region **194j** (denoted as having a diameter of V in FIG. 19) of the contralateral guidewire **194** can have a larger diameter than the first and second regions **194h, 194i.** For example, the diameter of the third region **194j** can be about 0.0174 inches with a tolerance 0.0002 inches. The third region **194j** can be longer than the first region **194h** and/or shorter than the second region **194i.** For example, the third region **194j** can have a length of 1.0 cm with a tolerance of 0.1 cm. A fourth region **194k** (denoted as having a diameter of K in FIG. 19) of the contralateral guidewire **194** can have a diameter greater than the first, second, and third regions **194h, 194i, 194j.** For example, the fourth region **194k** can have a diameter of about 0.0300 inches with a tolerance 0.0003 inches. A fifth region **1941** (denoted as having a diameter of H in FIG. 19) of the contralateral guidewire **194** can have a diameter that is less than the diameter of the fourth region **194k,** but greater than the diameter of the first, second, and third regions **194h, 194i, 194j.** For example, the diameter of the fifth region **194** can be about 0.0210 inches with a tolerance 0.0002 inches. The fifth region **194l** can be greater than each of the preceding regions **194h, 194i, 194j, 194k.** For example, the fifth region **194l** can have a length of 40.8 cm with a tolerance of 0.5 cm. A sixth region **194m** (denoted as having a diameter of U in FIG. 19) of the contralateral guidewire **194** can have a diameter that is about the same as the diameter of the third region **194j.** For example, the diameter of the sixth region **194m** can be about 0.0174 inches with a tolerance 0.0002 inches. The sixth region **194m** can be shorter than the can have a length of 0.8 cm with a tolerance of 0.1 cm. A seventh region **194n** (denoted as having a diameter of B in FIG. 19) of the contralateral guidewire **194** can have a diameter that is about the same as the second region **194i.** The diameter of the seventh region **194n** may provide the smallest diameter of the contralateral guidewire **194.** For example, the seventh region **194n** can have a diameter of 0.0080 inches with a tolerance 0.0002 inches. The seventh region can have a length of 5.2 cm with a tolerance of 0.1 cm. An eighth region **194o** (denoted as having a diameter of G in FIG. 19) of the contralateral guidewire **194** can have a diameter that is about the same as the diameter of the first region **194h.** For example, the eighth region **194o** can have a diameter of about 0.0160 inches with a tolerance 0.0002 inches. The eighth region **194o** can have a length of 0.5 cm with a tolerance of 0.1 cm. The contralateral guidewire **194** may include transition regions that soften diameter changes in the contralateral guidewire **194.** The transition regions may have a tapering angle that is formed between the longitudinal axis of the contralateral guidewire **194** and the outer wall of the transition region. The tapering angle of the transition regions can range between 10 and 60 degrees.

### Terminology

As will be recognized, certain embodiments described herein may be embodied within a form that does not provide all of the features and benefits set forth herein, as some features may be used or practiced separately from others. The scope of the inventions is indicated by the appended claims rather than by the foregoing description. All changes which come within the meaning and range of equivalency of the claims are to be embraced within their scope. For example, while the delivery system is described with respect to deploying a bifurcated stent in the abdominal aortic, it is further envisioned that the delivery system could be used to deliver a prosthesis having a main portion and at least one branch portion, or alternatively a prosthesis having only a straight, main branch portion, to other branched intravascular vessels (e.g., the thoracic aorta and a cardiac artery) and leave a guidewire positioned through the expanded prosthesis.

The term "guidewire" is to be interpreted broadly and may include, in addition to its ordinary and customary meaning to a person of ordinary skill in the art, any elongate member. Although the disclosure herein describes a locking assembly for reversibly coupling a guidewire to a delivery system, the locking assembly can also be used to reversibly couple any elongate structure to the delivery system, catheter, or otherwise.

As used herein, the relative terms "proximal" and "distal" shall be defined from the perspective of the delivery system. Thus, proximal refers to the direction of the control end of the delivery system and distal refers to the direction of the distal tip.

Note that the terms "first" and "second" branch portion can be used interchangeably and to refer to any branch vessel in the body, including but not limited to the ipsilateral vessel, the contralateral vessel, radial vessels, and subclavian vessels. Accordingly, in some embodiments, the "first" branch portion can refer to any branch portion including but not limited to the vessels set forth above. Similarly, the "second" branch portion can refer to any branch portion including but not limited to the vessels set forth below.

Conditional language, such as "can," "could," "might," or "may," unless specifically stated otherwise, or otherwise understood within the context as used, is generally intended to convey that certain embodiments include, while other embodiments do not include, certain features, elements, and/or steps. Thus, such conditional language is not generally intended to imply that features, elements, and/or steps are in any way required for one or more embodiments.

The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list.

The terms "approximately," "about," "generally," and "substantially" as used herein represent an amount close to the stated amount that still performs a desired function or achieves a desired result. For example, the terms "approximately", "about", and "substantially" may refer to an amount that is within less than 10% of the stated amount. As another example, in certain embodiments, the terms "substantially parallel" refer to a value, amount, or characteristic that departs from exactly parallel by less than or equal to 15 degrees.

The ranges disclosed herein also encompass any and all overlap, sub-ranges, and combinations thereof. Language such as "up to," "at least," "greater than," "less than," "between" and the like includes the number recited. Numbers preceded by a term such as "about" or "approximately" include the recited numbers. For example, "approximately 2 in." includes "2 in."

Some embodiments have been described in connection with the accompanying drawings. However, it should be understood that the figures are not drawn to scale. Distances, angles, etc. are merely illustrative and do not necessarily bear an exact relationship to actual dimensions and layout of the devices illustrated. Components can be added, removed, and/or rearranged. Further, the disclosure herein of any particular feature, aspect, method, property, characteristic, quality, attribute, element, or the like in connection with various embodiments can be used in all other embodiments set forth herein. Additionally, it will be recognized that any methods described herein may be practiced using any device suitable for performing the recited steps.

For purposes of this disclosure, certain aspects, advantages, and novel features are described herein. It is to be understood that not necessarily all such advantages may be achieved in accordance with any particular embodiment. Thus, for example, those skilled in the art will recognize that the disclosure may be embodied or carried out in a manner that achieves one advantage or a group of advantages as taught herein without necessarily achieving other advantages as may be taught or suggested herein.

Moreover, while illustrative embodiments have been described herein, the scope of any and all embodiments having equivalent elements, modifications, omissions, combinations (e.g., of aspects across various embodiments), adaptations and/or alterations as would be appreciated by those in the art based on the present disclosure. The limitations in the claims are to be interpreted broadly based on the language employed in the claims and not limited to the examples described in the present specification or during the prosecution of the application, which examples are to be construed as non-exclusive. Further, the actions of the disclosed processes and methods may be modified in any manner, including by reordering actions and/or inserting additional actions and/or deleting actions. It is intended, therefore, that the specification and examples be considered as illustrative only, with a true scope being indicated by the claims.

Any methods disclosed herein need not be performed in the order recited. The methods disclosed herein include certain actions taken by a practitioner; however, they can also include any third-party instruction of those actions, either expressly or by implication. For example, actions such as "releasing the delivery system from the locked configuration" include "instructing release of the delivery system from the locked configuration."

## Claims

1. A locking assembly (300, 300', 300") for releasably coupling a contralateral guidewire (194) to an ipsilateral catheter, the locking assembly comprising:
an anchoring portion (308) configured to engage the ipsilateral catheter; and
an interlock portion (346, 346', 346") configured to retain a distal portion (194b) of the contralateral guidewire when the contralateral guidewire is advanced or retracted unless a vertical force is applied to the contralateral guidewire by a release member (311) contacting with the locking assembly and applying the vertical force to draw the distal portion (194b) out of the interlock portion (346, 346', 346").

2. The locking assembly (300, 300', 300") of claim 1, wherein the interlock portion (346, 346', 346") comprises a lumen (312, 312', 312"), the lumen being shaped to retain the contralateral guidewire (194) when the contralateral guidewire is retracted.

3. The locking assembly (300, 300', 300") of claim 2, wherein the lumen (312, 312', 312") comprises a distal portion (374'), a proximal portion (483'), and an intermediate portion (382') therebetween, a diameter of the intermediate portion being less than a diameter of the distal portion and a diameter of the proximal portion.

4. The locking assembly (300, 300', 300") of claim 1, further comprising a retention member (334) configured to frictionally retain the contralateral guidewire (194) when the contralateral guidewire is advanced.

5. The locking assembly (300, 300', 300") of claim 4, wherein the retention member (334) comprises an elastomeric material.

6. The locking assembly of (300, 300', 300") claim 4, wherein the retention member (334) comprises an opening (348) configured to receive the contralateral guidewire.

7. The locking assembly of (300, 300', 300") claim 1, wherein the interlock portion (346, 346', 346") is configured to release the contralateral guidewire (194) when a force is applied to an intermediate portion of the contralateral guidewire at an angle of less than or equal to about 60 degrees from the distal portion of the contralateral guidewire in the interlock portion.

## Patentansprüche

1. Verriegelungsanordnung (300, 300', 300'') zum lösbaren Koppeln eines kontralateralen Führungsdrahts (194) an einen ipsilateralen Katheter, wobei die Verriegelungsanordnung umfasst:
einen Verankerungsabschnitt (308), der dazu konfiguriert ist, den ipsilateralen Katheter in Eingriff zu nehmen; und
einen Interlock-Abschnitt (346, 346', 346''), der dazu konfiguriert ist, einen distalen Abschnitt (194b) des kontralateralen Führungsdrahts festzuhalten, wenn der kontralaterale Führungsdraht vorgeschoben oder zurückgezogen wird, es sei denn, eine vertikale Kraft wird auf den kontralateralen Führungsdraht ausgeübt, indem ein Löseelement (311) in Kontakt mit der Verriegelungsanordnung kommt und die vertikale Kraft ausübt, um den distalen Abschnitt (194b) aus dem Interlock-Abschnitt (346, 346', 346'') herauszuziehen.

2. Verriegelungsanordnung (300, 300', 300'') nach Anspruch 1, wobei der Interlock-Abschnitt (346, 346', 346'') ein Lumen (312, 312', 312'') umfasst, wobei das Lumen so geformt ist, dass es den kontralateralen Führungsdraht (194) festhält, wenn der kontralaterale Führungsdraht zurückgezogen wird.

3. Verriegelungsanordnung (300, 300', 300'') nach Anspruch 2, wobei das Lumen (312, 312', 312'') einen distalen Abschnitt (374'), einen proximalen Abschnitt (483') und einen Zwischenabschnitt (382') dazwischen umfasst, wobei ein Durchmesser des Zwischenabschnitts weniger als ein Durchmesser des distalen Abschnitts und ein Durchmesser des proximalen Abschnitts ist.

4. Verriegelungsanordnung (300, 300', 300'') nach Anspruch 1, ferner umfassend ein Halteelement (334), das dazu konfiguriert ist, den kontralateralen Führungsdraht (194) reibschlüssig festzuhalten, wenn der kontralaterale Führungsdraht vorgeschoben wird.

5. Verriegelungsanordnung (300, 300', 300'') nach Anspruch 4, wobei das Halteelement (334) ein elastomeres Material umfasst.

6. Verriegelungsanordnung (300, 300', 300'') nach Anspruch 4, wobei das Halteelement (334) eine Öffnung (348) umfasst, die dazu konfiguriert ist, den kontralateralen Führungsdraht aufzunehmen.

7. Verriegelungsanordnung (300, 300', 300'') nach Anspruch 1, wobei der Interlock-Abschnitt (346, 346', 346'') dazu konfiguriert ist, den kontralateralen Führungsdraht (194) zu lösen, wenn eine Kraft auf einen Zwischenabschnitt des kontralateralen Führungsdrahts unter einem Winkel von weniger als oder gleich etwa 60 Grad von dem distalen Abschnitt des kontralateralen Führungsdrahts in dem Interlock-Abschnitt ausgeübt wird.

## Revendications

1. Ensemble de verrouillage (300, 300', 300'') pour le couplage de façon amovible d'un fil-guide controlatéral (194) à un cathéter ipsilatéral, l'ensemble de verrouillage comprenant :
une partie d'ancrage (308) configurée pour venir en prise avec le cathéter ipsilatéral ; et
une partie de verrouillage (346, 346', 346'') configurée pour retenir une partie distale (194b) du fil-guide controlatéral lorsque le fil-guide controlatéral est avancé ou rétracté, à moins qu'une force verticale ne soit appliquée au fil-guide controlatéral par un élément de libération (311) venant en contact avec l'ensemble de verrouillage et appliquant la force verticale pour tirer la partie distale (194b) hors de la partie de verrouillage (346, 346', 346").

2. Ensemble de verrouillage (300, 300', 300'') selon la revendication 1, dans lequel la partie de verrouillage (346, 346', 346'') comprend une lumière (312, 312', 312"), la lumière étant conformée pour retenir le fil-guide controlatéral (194) lorsque le fil-guide controlatéral est rétracté.

3. Ensemble de verrouillage (300, 300', 300'') selon la revendication 2, dans lequel la lumière (312, 312', 312'') comprend une partie distale (374'), une partie proximale (483') et une partie intermédiaire (382') entre celles-ci, un diamètre de la partie intermédiaire étant inférieur à un diamètre de la partie distale et à un diamètre de la partie proximale.

4. Ensemble de verrouillage (300, 300', 300'') selon la revendication 1, comprenant en outre un élément de retenue (334) configuré pour retenir par friction le fil-guide controlatéral (194) lorsque le fil-guide controlatéral est avancé.

5. Ensemble de verrouillage (300, 300', 300'') selon la revendication 4, dans lequel l'élément de retenue (334) comprend un matériau élastomère.

6. Ensemble de verrouillage (300, 300', 300'') selon la revendication 4, dans lequel l'élément de retenue (334) comprend une ouverture (348) configurée pour recevoir le fil-guide controlatéral.

7. Ensemble de verrouillage (300, 300', 300'') selon la revendication 1, dans lequel la partie de verrouillage (346, 346', 346'') est configurée pour libérer le fil-guide controlatéral (194) lorsqu'une force est appliquée à une partie intermédiaire du fil-guide controlatéral selon un angle inférieur ou égal à environ 60 degrés par rapport à la partie distale du fil-guide controlatéral située dans la partie de verrouillage.
